# EUROPEAN PATENT SPECIFICATION

(11) **EP 2 583 674 B1**
(45) Date of publication and mention of the grant of the patent: **18.11.2015**
(21) Application number: 11790010.0
(22) Date of filing: 01.06.2011
(51) Int. Cl.: A61K 9/22, A61K 9/20, A61K 47/38, A61K 31/216, A61K 9/24

(54) **ACECLOFENAC SLOW-RELEASE PREPARATION PROVIDING AN OPTIMUM PHARMACOLOGICAL CLINICAL EFFECT WHEN ADMINISTERED ONCE A DAY**
LANGSAM FREIGESETZTES ACECLOFENAC-PRÄPARAT MIT OPTIMALER PHARMAKOLOGISCHER WIRKUNG BEI EINMAL PRO TAG ERFOLGENDER VERABREICHUNG
PRÉPARATION D'ACÉCLOFÉNAC À LIBÉRATION LENTE PRÉSENTANT UN EFFET CLINIQUE PHARMACOLOGIQUE OPTIMAL LORSQU'ELLE EST ADMINISTRÉE UNE FOIS PAR JOUR

(30) Priority: 01.06.2010 KR 20100052030
(43) Date of publication of application: 24.04.2013
(73) Proprietor: Korea United Pharm, Inc., Seoul 135-010 (KR)
(72) Inventor: LEE, Beom Jin, Seoul 138-773 (KR); JUNG, Won-Tae, Seoul Korea Seoul 135-010 (KR); CHOI, Youn Woong, Ansan-si Gyeonggi-do 425-022 (KR); NAM, Kyu Yeol, Seoul 134-703 (KR); CHO, Sang-Min, Siheung-si Gyeonggi-do 429-450 (KR); JANG, Jae Sang, Incheon 406-050 (KR); CHOI, Min-Ji, Seoul 151-050 (KR)
(74) Representative: Katzameyer, Michael
(86) International application number: PCT/KR2011/003989
(87) International publication number: WO 2011/152652

(56) References cited:
- WO-A1-2010/090371
- CN-A- 101 108 170
- KR-A- 20030 003 500
- KR-B1- 100 806 541
- KR-B1- 100 812 538
- KARTHIKEYINI S C ET AL: "Formulation and evaluation of aceclofenac sodium bilayer sustained release tablets", INTERNATIONAL JOURNAL OF CHEMTECH RESEARCH, SPHINX KNOWLEDGE HOUSE, INDIA, vol. 1, no. 4, 1 January 2009 (2009-01-01) , pages 1381-1385, XP009162303, ISSN: 0974-4290
- C Setty: "Development of Fast Dispersible Aceclofenac Tablets: Effect of Functionality of Superdisintegrants", Indian J Pharm Sci, 2 March 2008 (2008-03-02), pages 180-185, XP055117791, Internet DOI: 10.4103/0250-474X.41452 Retrieved from the Internet: URL:http://www.ncbi.nlm.nih.gov/pmc/articl es/PMC2792493/ [retrieved on 2014-05-13]
- GHOSH, S. ET AL.: 'Preparation and evaluation of aceclofenac sustained release formulation and comparison of formulated and marketed product' INT. J. MED. MEDICAL SCIENCES vol. 1, no. 9, 2009, pages 375 - 382, XP055108024

## Description

### [Technical Field]

The present invention relates to a slow-release tablet improving release of an ideal drug showing an almost linear release profile by solubilizing and controlled-releasing an insoluble component such as aceclofenac, diclofenac or a pharmaceutically available salt thereof, and expressing an optimum pharmacological clinical effect when orally administered to a patient by enhancing bioavailability according to an improved dissolution rate. Particularly, the present invention relates to a controlled-release oral preparation composed of an immediate-release layer containing aceclofenac and a slow-release layer, which uniformly and meticulously controls drug release.

The present invention increases solubility of insoluble aceclofenac under an acidic condition (gastric juice) by adding a solubilizer having fast pain-killing and anti-inflammatory effects, and shows a consistent pharmacological activity for 24 hours by controlling the release of aceclofenac using a polymer for release control. Aceclofenac is a drug which is unstable in an acid and not soluble in the stomach. Therefore, to overcome these problems, in the present invention, a dissolution rate of aceclofenac under an acidic condition was enhanced by adding a solubilizer to an immediate-release layer. In addition, the slow-release layer includes a pH-independent polymer base, a pH-dependent polymer base and an excipient. Here, the pH-independent polymer base absorbs moisture in oral administration to control drug release using a water-soluble matrix system, and the pH-dependent polymer base controls the drug release in a pH-dependent way. As a result, the present invention provides an aceclofenac slow-release preparation meticulously controlling release of aceclofenac until it reaches the intestines from the stomach, and particularly remarkably improving controlled-release of the drug in the intestines.

To have a fast clinical pharmacological activity, in an *in-vitro* test, 45 to 65% of drugs should be released within an hour, and to have an effective clinical pharmacological activity for 24 hours, a controlled-release rate should be 65 to 85% within 12 hours, or 85% or more within 24 hours.

It could be seen that the initial increase in release of aceclofenac according to an amount of a solubilizer added in the *in-vitro* test was similar to an initial increase in bioavailability in a pre-clinical test. Such a result proves that the elusion result and the degree of solubilization of aceclofenac in the *in-vitro* test have a close relationship with bioavailability.

Moreover, in the *in vitro* test, the maximum concentration in blood (Cₘₐₓ) of aceclofenac is 30 to 60 µg/ml, the area under the blood concentration versus time curve (AUCₜ) is 5 to 15 h*µg/ml and 30 to 60 h* µg /ml (respectively) and the time to peak concentration (Tₘₐₓ) is 1.2 to 3 h.

In short, the present invention is associated with development of a controlled-release preparation orally administered once a day to express an optimum pharmacological clinical effect. Here, the controlled-release preparation is composed of a bilayered tablet, a dual tablet and a multilayered tablet including an immediate-release layer containing aceclofenac, a solubilizer, a water-soluble additive, a short-acting excipient, a disintegrating agent and a filler, and a slow-release layer containing aceclofenac, a solubilizer, a binder, a base for controlling release and a lubricant.

### [Background Art]

Aceclofenac (2-[(2,6-dichlorophenyl)amino]-phenylacetoxyacetic acid) is a known compound having the following Formula 1, which is an anti-inflammatory painkilling drug of a phenylacetic acid series showing a remarkable effect on toothaches, and pains after surgery or childbirth as well as chronic articular diseases such as rheumatic arthritis, osteoarthropathy or ankylosing spondylitis. The drug (aceclofenac) has an excellent therapeutic effect because the drug easily penetrates into inflammatory tissue such as a joint and thus has an excellent effect of preventing generation of prostaglandin in the inflammatory tissue, compared to an inflammatory painkilling agent such as naproxen or diclofenac. On the other hand, aceclofenac has a weak effect of preventing generation of normal prostaglandin in the stomach mucosa, and thereby reduces gastroenteric problems. Therefore, aceclofenac is suitable for long-term administration, and prevents deterioration of rheumatoid arthritis or osteoarthritis since it particularly inhibits generation of interleukin destroying cartilage in a joint so as to stimulate generation of glycosaminoglycan, which is a component of articular cartilage.

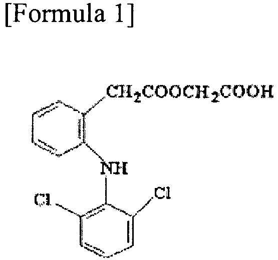

Aceclofenac is easily dissolved in an organic solvent, but relatively less dissolved in water. Aceclofenac is absorbed fast in the gastrointestinal tract when orally administered, and distributed at a high concentration in the kidneys, urinary bladder, liver or thyroid gland and at a low concentration in the eyes, brain or lipid tissue. When aceclofenac is orally administered, an onset time is within 30 minutes, a time to Cₘₐₓ (Tₘₐₓ) is approximately 1.5 to 2.5 hours, and duration is approximately 12 hours. In the oral administration, aceclofenac is included at 46 to 75% of an administered drug at Tₘₐₓ. After Tₘₐₓ, a large extent of metabolism of aceclofenac is performed. Among 9 of metabolites, 4-hydroxyaceclofenac, 4-hydroxydiclofenac and diclofenac are main metabolites, and the others are usually included at less than 1 to 2%, although this differs for the individual.

Aceclofenac is metabolized into 4-hydroxyaceclofenac, 5-hydroxyaceclofenac, 4-hydroxydiclofenac, 5-hydroxydiclofenac and other metabolites after it is absorbed in the body as a prodrug-type drug, and takes effect due to diclofenac and 4-hydroxydiclofenac which are the main active metabolites thereof.

Afterward, approximately 70% of aceclofenac and the metabolites thereof in the body are excreted through urine and approximately 20% thereof through stool. It is known that 4-hydroxydiclofenac and 4-hydroxyaceclofenac are excreted at high rates through urine, which is the main excretion route, and an excretion rate (half-life) is approximately 4 hours.

Clinical characteristics of aceclofenac include: 1) suitability for rheumatoid arthritis or osteoarthritis since aceclofenac inhibits the generation of interleukin-1 destroying articular cartilage and stimulates the generation of glycosaminoglycan, which is a component of articular cartilage; 2) minimization of gastroenteric problems since aceclofenac has less of an effect on the generation of normal prostaglandin in the gastric mucosa; and 3) inhibition of the generation of prostaglandin in lesions because the drug penetrates into inflammatory lesions such as joints at a high concentration.

Today, aceclofenac is developed and commercially available in the form of a tablet or soluble soft capsule. 100 mg of aceclofenac is administered in the form of a tablet once a day, or a capsule twice a day. However, while aceclofenac should be administered for a long time due to the characteristics of musculoskeletal patients, a formulation capable of being administered once a day has not been developed, which has caused inconvenience to patients to whom aceclofenac is administered for a long time in terms of the guidelines for the administration of drugs and patient compliance. The present invention solves the problems caused when aceclofenac is administered twice a day, and therefore has advantages in terms of the administration of drugs and the therapeutic effect in musculoskeletal patients.

In non-steroidal anti-inflammatory drugs (NSAIDs), due to pharmacological characteristics, early expression of the therapeutic effects is important. A general slow-release tablet developed by the conventional technique has long lasting effects for about 24 hours, but is difficult to reach an effective blood concentration in the beginning. In addition, when rapid pharmacological activities are expressed in the beginning after oral administration, the effective pharmacological activities may not last for 24 hours. Accordingly, in the formulation satisfying the expression of rapid pharmacological activities and the lasting of therapeutic effects at the same time, it is important that an early dissolution rate be shown quickly in a bilayered tablet, a dual tablet and a multilayered tablet (the formulation composed of an immediate-release layer and a slow-release layer) until the predetermined point in time, and the effective blood concentration of the drug be uniformly maintained for 24 hours.

While the dissolution rate was controlled using hydroxypropylmethylcellulose (HPMC) and a carbomer which are a pH-dependent polymer and a pH-independent polymer, respectively, the HPMC and carbomer have an disadvantage that the dissolution of aceclofenac is difficult to regulate meticulously in the stomach, which is in an acidic condition, only using the carbomer controlling the dissolution of aceclofenac in an alkali solution and HPMC maintaining a matrix form of a tablet. Therefore, in the present invention, a preparation that maintains the pharmacological activities for 24 hours using a slow-release base to enhance bioavailability and shows lasting effects by increasing solubility in gastric juice using a solubilizer to express rapid pharmacological activities of aceclofenac is disclosed.

Karthikeyini et al discloses a bilayer tablet for aceclofenac sodium.

### [Disclosure]

### [Technical Problem]

The present invention is directed to providing an aceclofenac-containing slow-release tablet, which is formulated into a multilayered tablet composed of an immediate-release layer and a slow-release layer and having aceclofenac as an effective component. Due to the tablet, 100 mg of aceclofenac, which has normally been administered twice a day, can be administered once a day. Moreover, the tablet rapidly reaches an effective blood concentration in the beginning of the administration of an anti-inflammatory drug and consistently maintains the effective blood concentration, which is the most important characteristic and thus improves therapeutic effects, and enhances the convenience of administration of the drug and compliance in patients by simplifying administration therapy for musculoskeletal patients.

### [Technical Solution]

The present application discloses an aceclofenac controlled-release preparation for oral administration, comprising: an immediate-release layer including aceclofenac, a solubilizer, a water-soluble additive, a disintegrating agent, a filler and a fast-acting excipient and a slow-release layer including aceclofenac, a solubilizer and a base for release control, wherein the base for release control uses a mixture of hydroxypropylmethylcellulose (HPMC) and a carbomer in a weight ratio of 1: to 30:1, and the preparation is administered once a day.

In one aspect, the present invention provides a slow-release tablet for oral administration, which is formed of a bilayered or multilayered tablet composed of an immediate-release layer including aceclofenac, a solubilizer, a water-soluble additive, a disintegrating agent, a filler and a fast-acting excipient and a slow-release layer including aceclofenac, a solubilizer and a base for release control, and includes a lubricant. The tablet may further and selectively include a binder.

In one exemplary embodiment of the present invention, a controlled-release-type oral preparation containing aceclofenac includes an immediate-release layer including aceclofenac, a solubilizer, a water-soluble additive, a disintegrating agent, a filler and a fast-acting excipient, and a slow-release layer including aceclofenac, a solubilizer and a base for release control. As the base for release control, HPMC and a carbomer are mixed in a ratio of 1:1 to 30: 1. The preparation is administered once a day.

In another exemplary embodiment of the present invention, HPMC may have a viscosity of 80,000 to 120,000 cps.

In still another exemplary embodiment of the present invention, the immediate-release layer may contain aceclofenac in a content of 40 to 100 mg, and the slow-release layer may contain aceclofenac in a content of 100 to 160 mg.

In yet another exemplary embodiment of the present invention, the preparation may use at least one selected from the group consisting of a poloxamer, organic acid and organic acid salt monoglyceride derivatives, a glyceride and a derivative thereof, diethylene glycol ester, sorbital ester, a carbonate salt and polyoxyethylene ether, Gelucire 44/14, sodium hydrocarbons of polyethyleneglycols, Brijs and Labrafils, sodium laurylsulfate, and a mixture thereof as a solubilizer.

In yet another exemplary embodiment of the present invention, the preparation shows a control release rate of 45 to 65% for 1 hour, 65 to 85% for 12 hours, and 85% or more for 24 hours in an *in-vitro* test in which a paddle revolves at 50 rpm/min according to the guidelines for general tests regulated by Korean Pharmacopoeia. Here, the immediate-release layer is coated on a surface of the slow-release layer.

In yet another exemplary embodiment of the present invention, the preparation is a pellet-type tablet or multi tablet, which is prepared by mixing an immediate-release layer and a slow-release layer.

In yet another exemplary embodiment of the present invention, the preparation is a capsule filled with a mixture of an immediate-release layer and a slow-release layer.

In yet another exemplary embodiment of the present invention, in an *in-vivo* test using healthy persons, the preparation has a maximum blood concentration (Cₘₐₓ) of aceclofenac of 30 to 60 µg/ml, an area under blood concentration versus time curve (AUCₜ) is 5 to 15 h*µg/ml and 30 to 60 h*µg/ml (respectively) and a time to peak concentration (Tₘₐₓ) is 1.2 to 3 h.

### [Advantageous Effects]

A tablet for oral administration formed in a bilayered structure including an immediate-release layer and a slow-release layer according to the present invention shows fast pain killing and anti-inflammatory effects, and consistently maintains an active component and effective active metabolites at uniform concentrations in the serum for a considerable time when orally administered, thereby reducing administration frequency, increasing drug adaptability with respect to patients, and thus increasing compliance.

### [Description of Drawings]

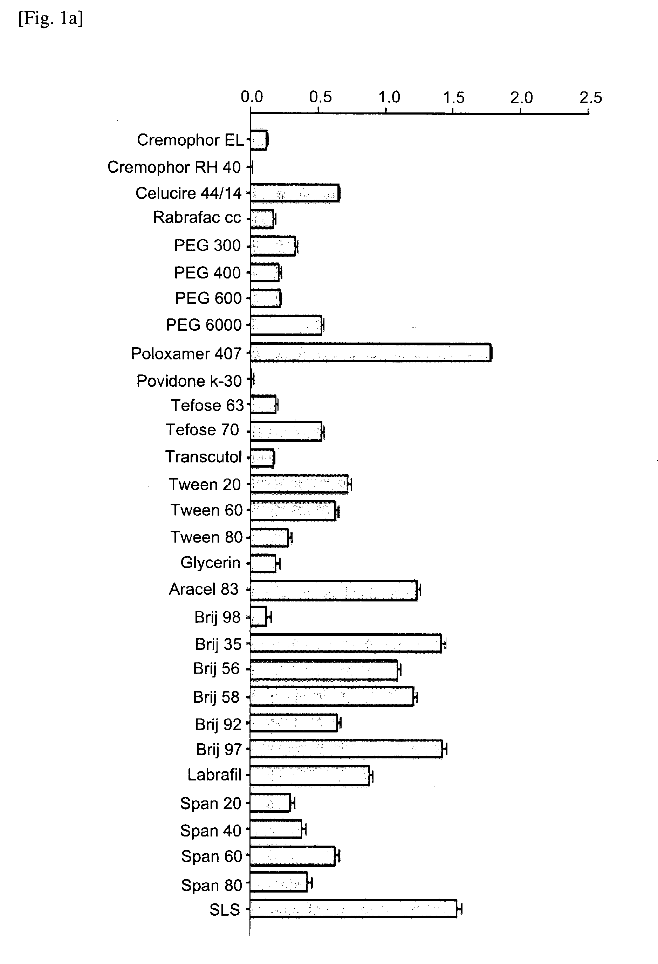

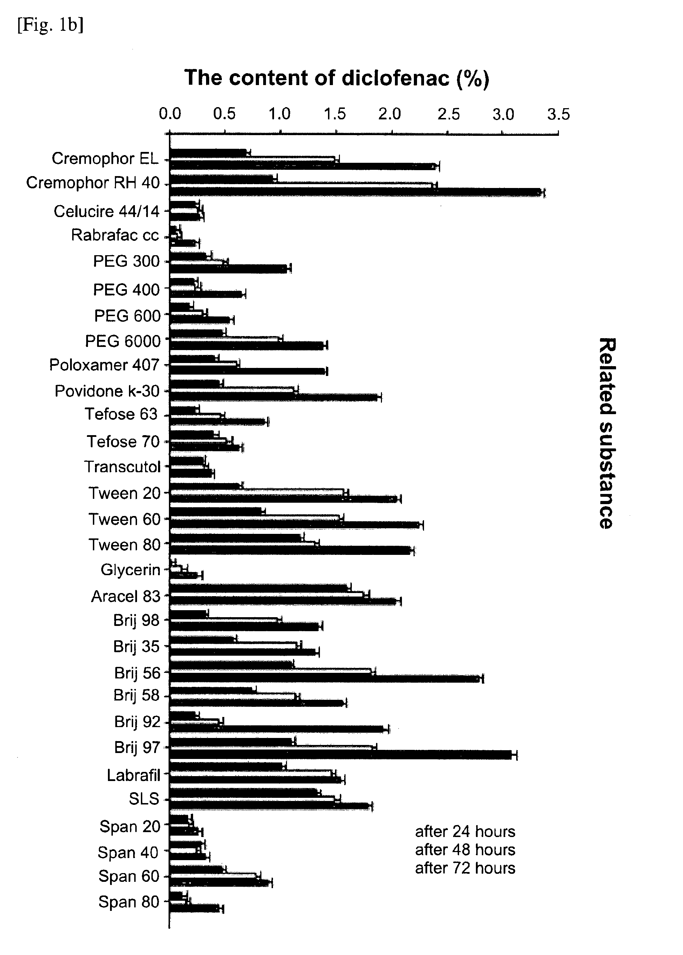

FIGS. 1A and 1B are graphs of solubility of aceclofenac and a content of a softening substance (e.g., diclofenac) depending on respective kinds of solubilizers;

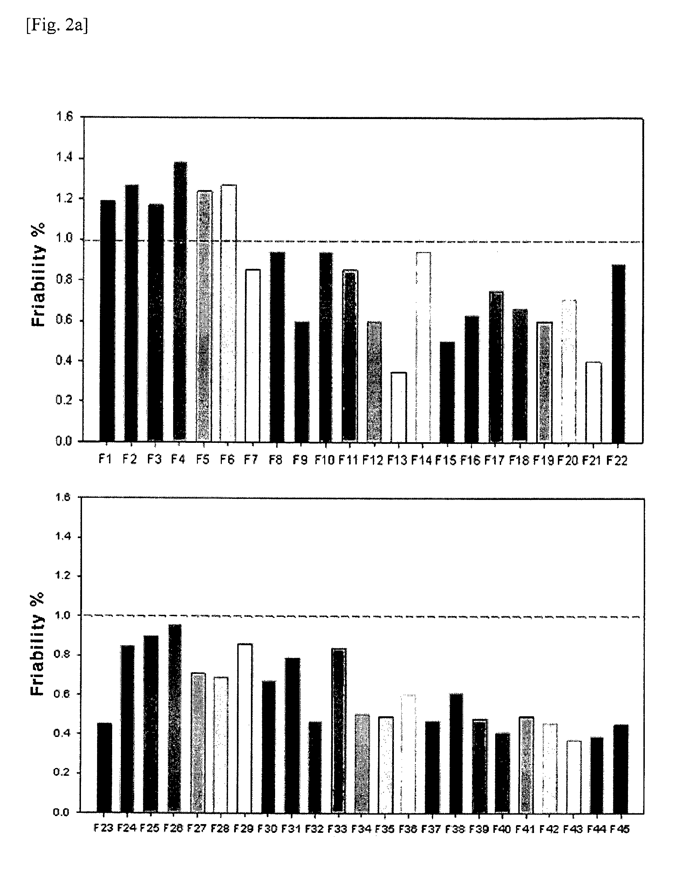

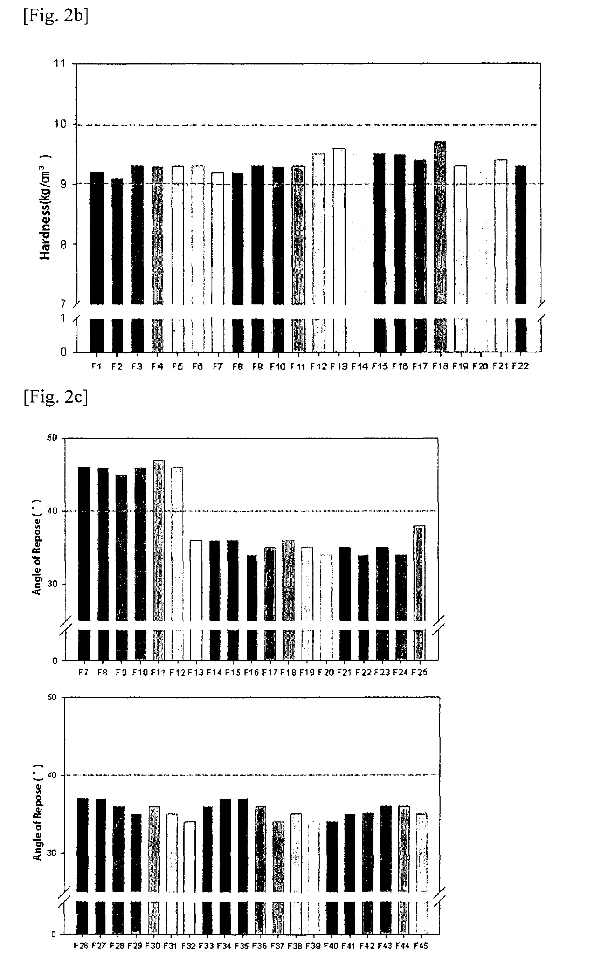

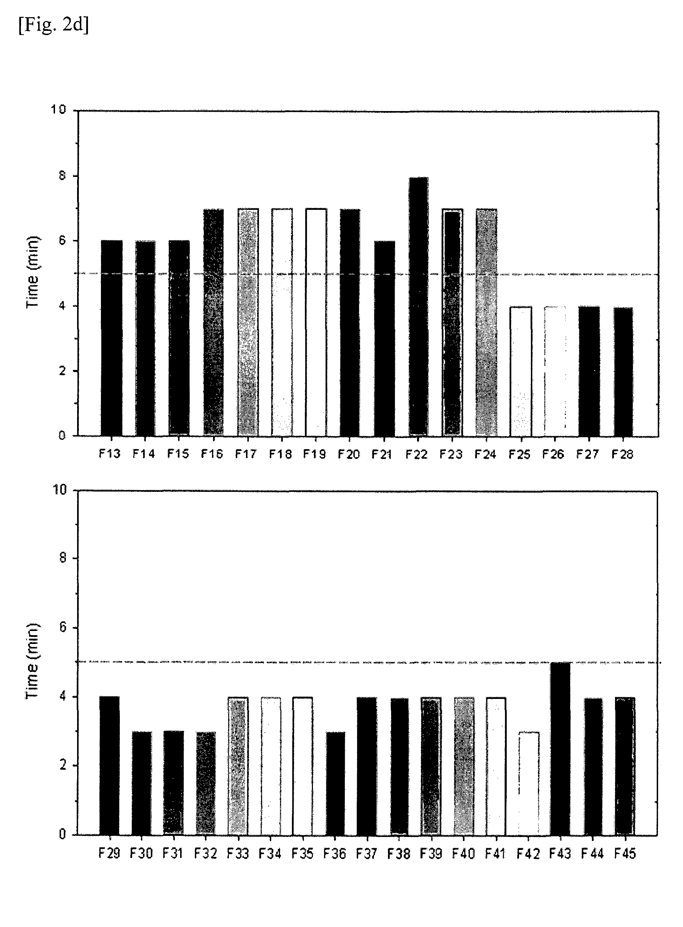

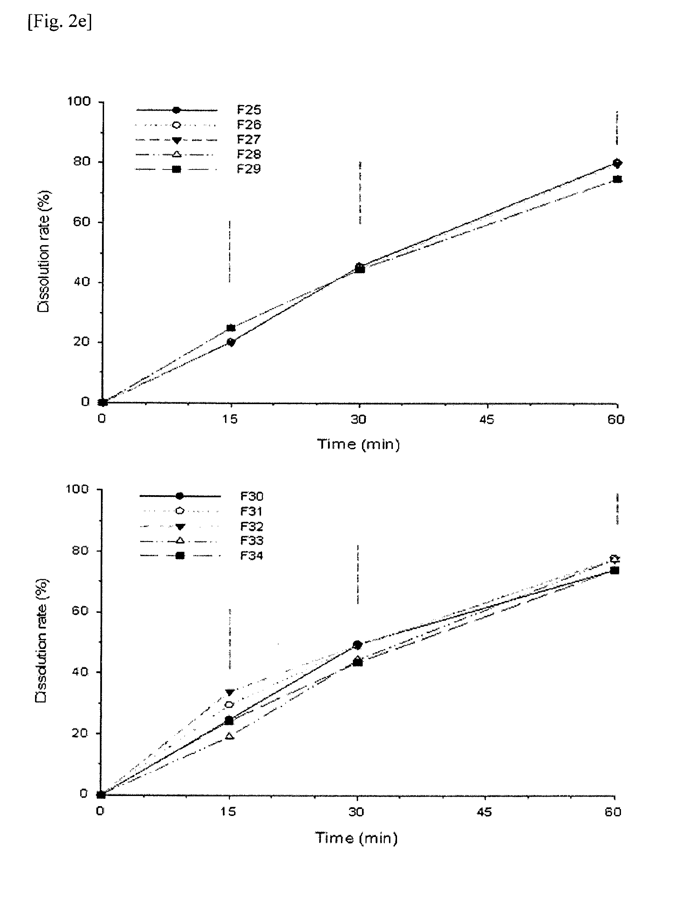

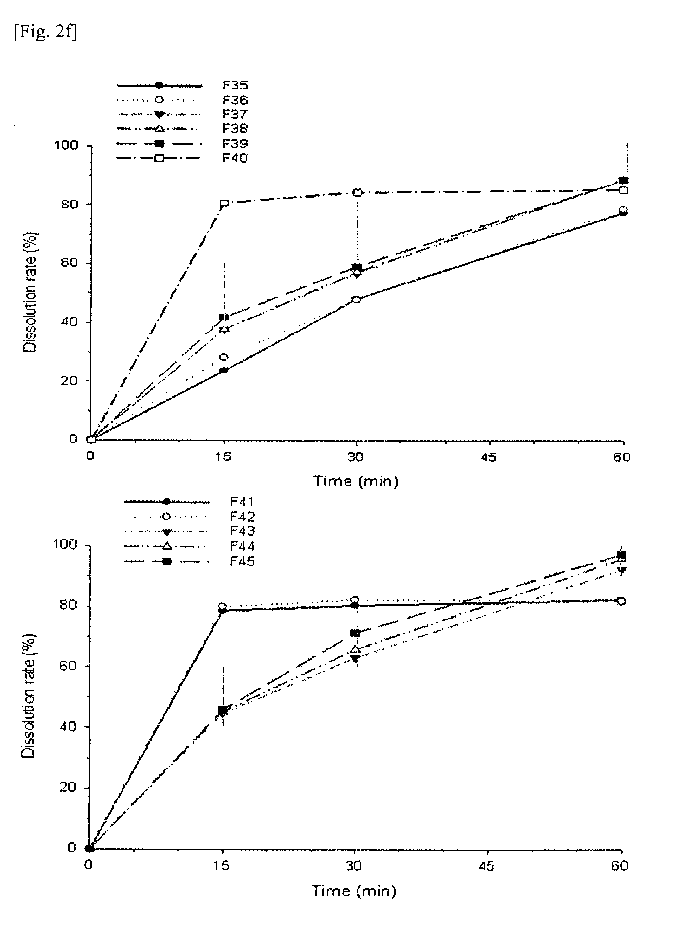

FIGS. 2A to 2F are graphs showing test results of friability, hardness, flowability, disintegrability and dissolution of aceclofenac depending on an excipient included in an immediate-release layer part, which are shown in Table 4, according to the present invention;

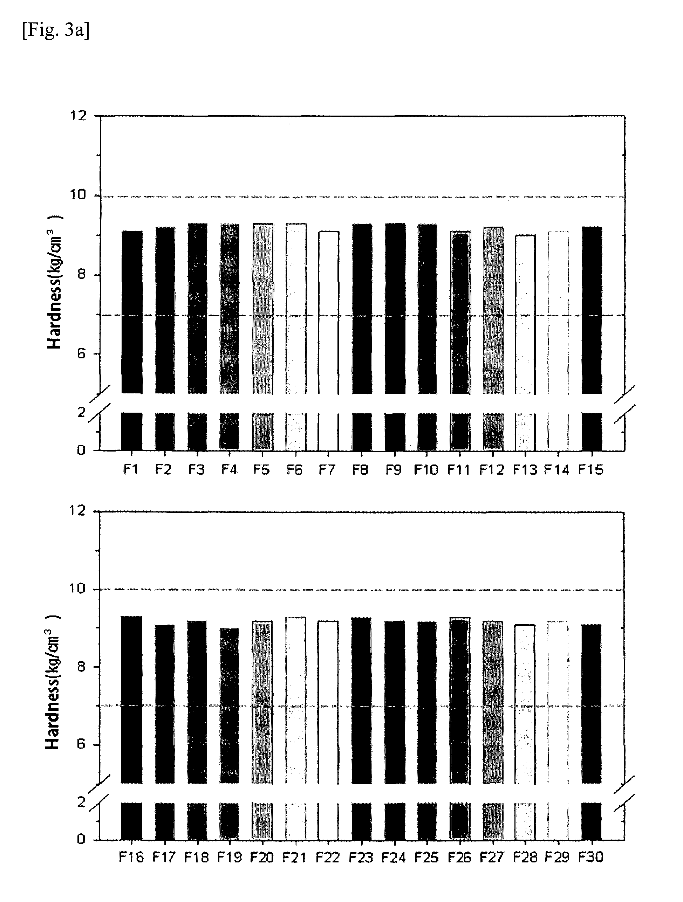

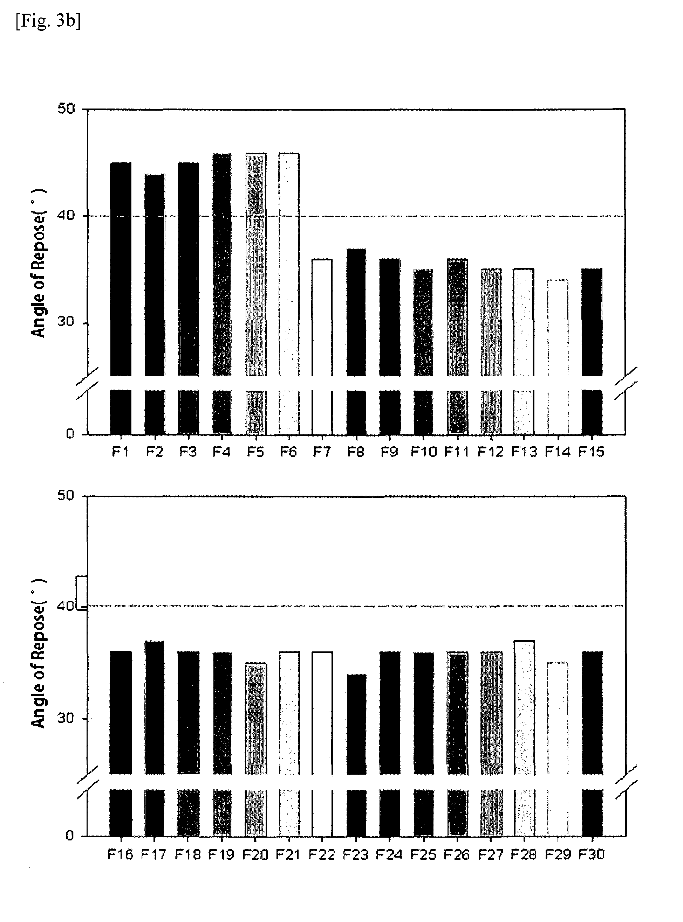

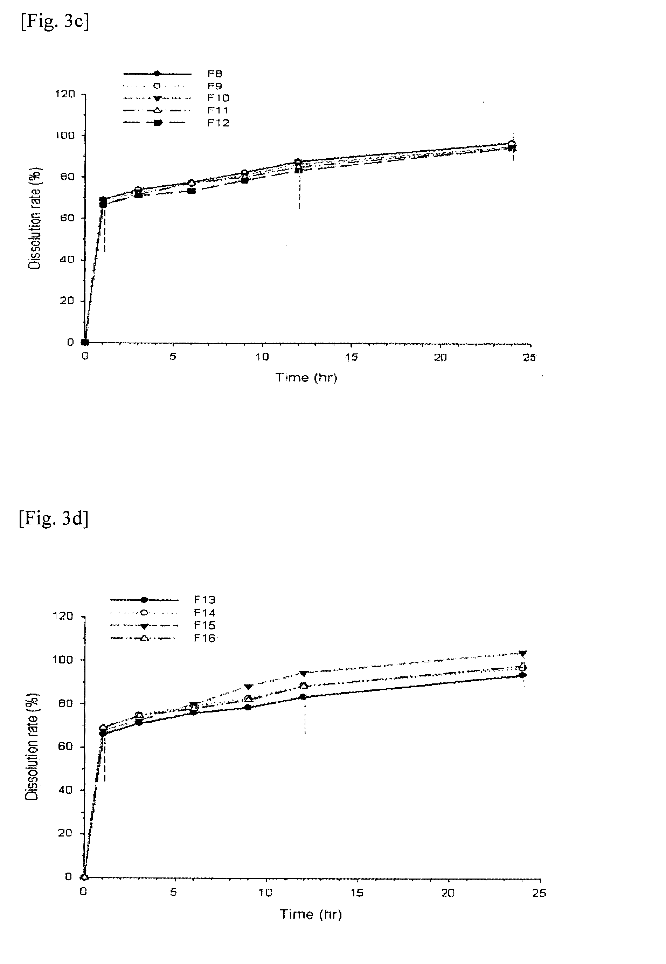

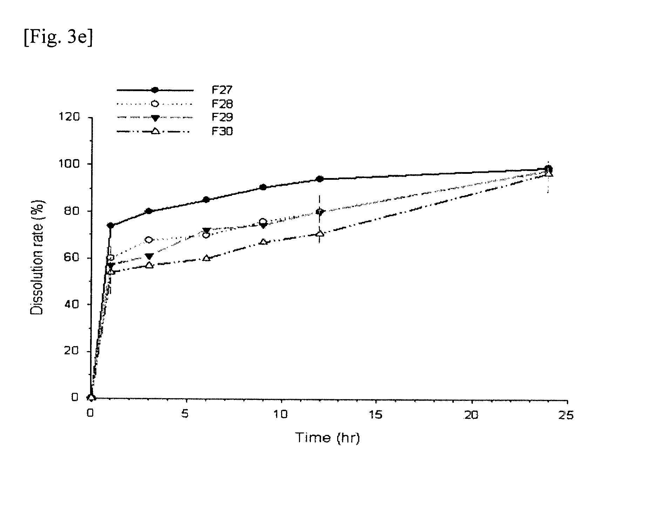

FIGS. 3A to 3E are graphs showing results of hardness, flowability and dissolution tests depending on the formulation of an immediate-release layer determined in Experimental Example 3 and the formulation of a slow-release layer shown in Table 5;

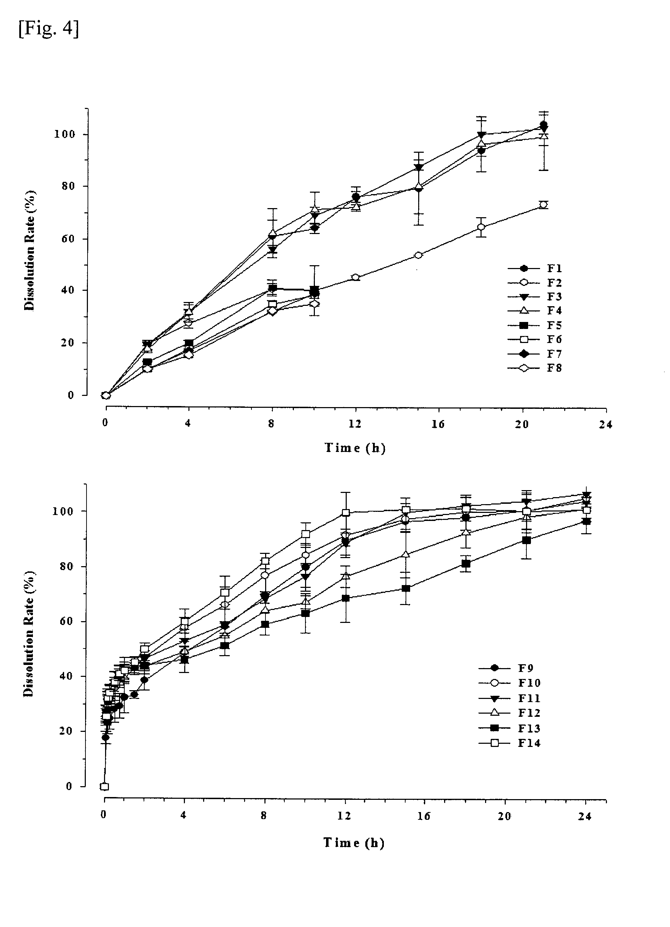

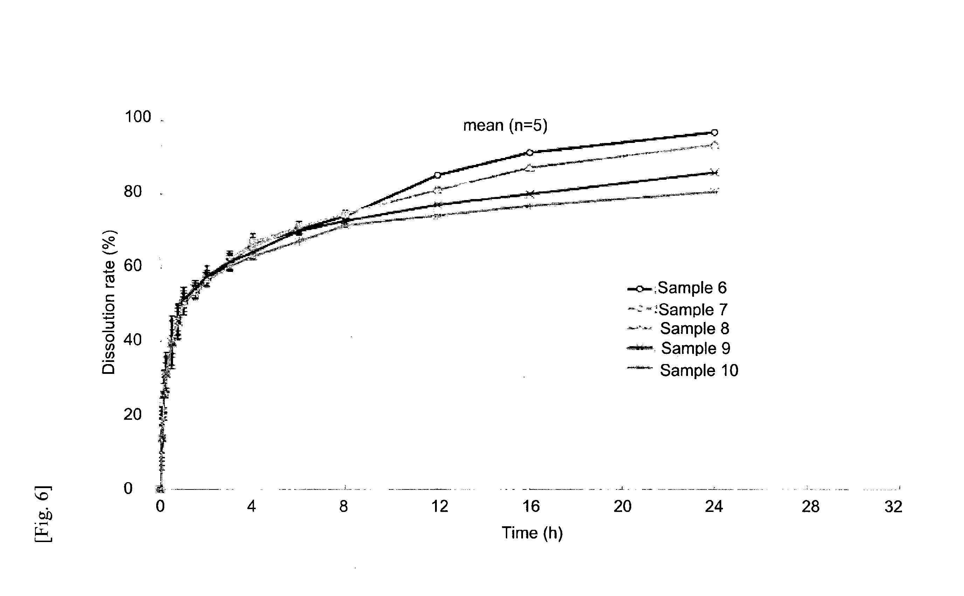

FIG. 4 is a graph showing results of a dissolution rate test for a preparation depending on the formulations of an immediate-release layer and a slow-release layer shown in FIG. 6 according to the present invention;

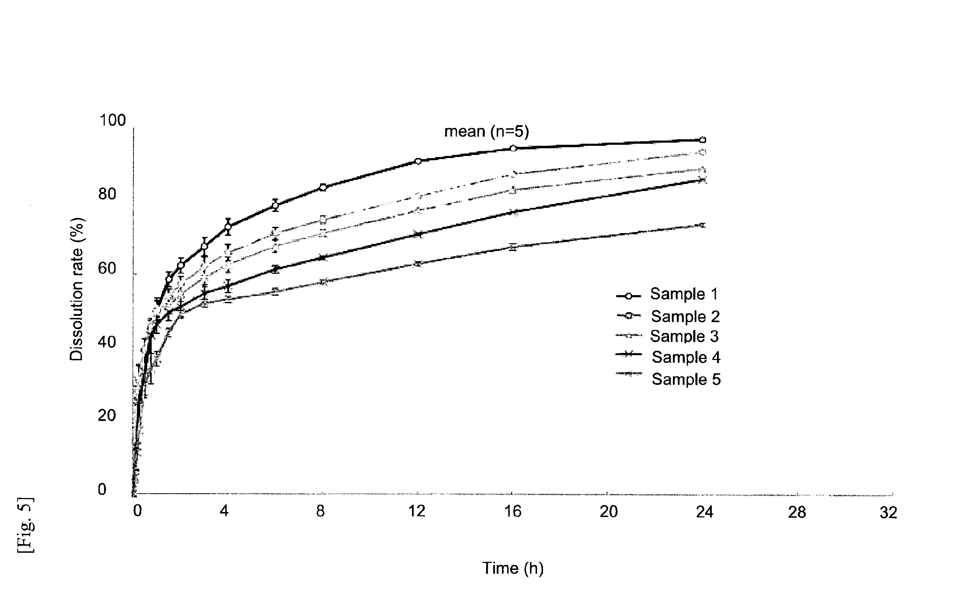

FIG. 5 is a graph showing results of a dissolution rate test for an aceclofenac slow-release preparation depending on the content of HPMC shown in Table 7 according to the present invention;
FIG. 6 is a graph showing results of a dissolution rate test for an aceclofenac slow-release preparation depending on the content of a carbomer shown in Table 8 according to the present invention;

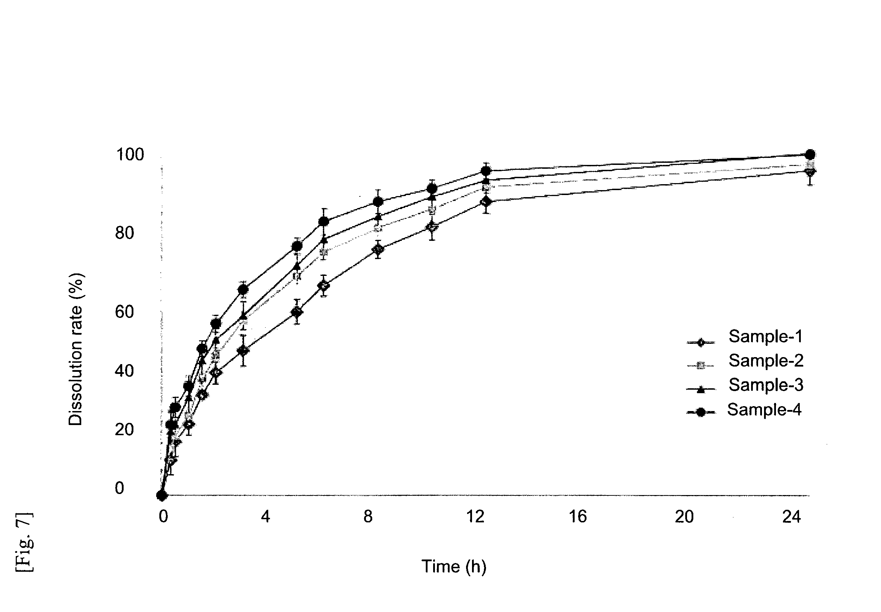

FIG. 7 is a graph showing results of a dissolution rate test for an aceclofenac slow-release preparation depending on the contents of aceclofenac contained in an immediate-release layer and a slow-release layer shown in Table 9 according to the present invention;

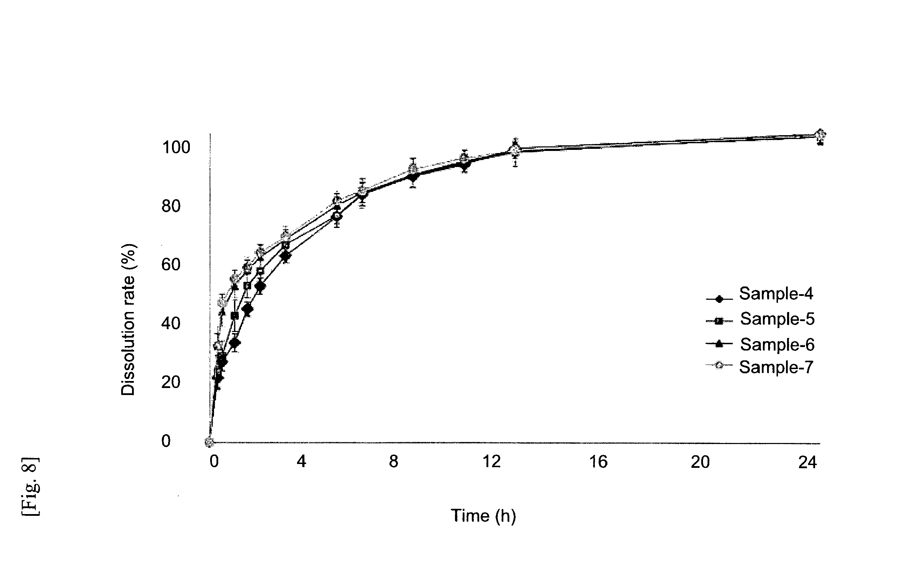

FIG. 8 is a graph showing results of a dissolution rate test for an aceclofenac slow-release preparation depending on the content of a solubilizer shown in Table 10 according to the present invention;

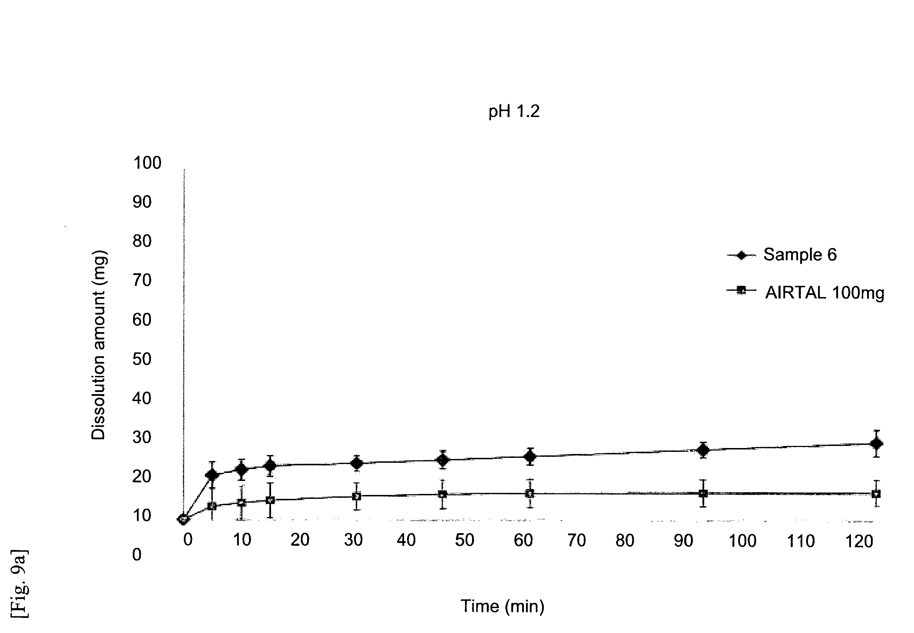

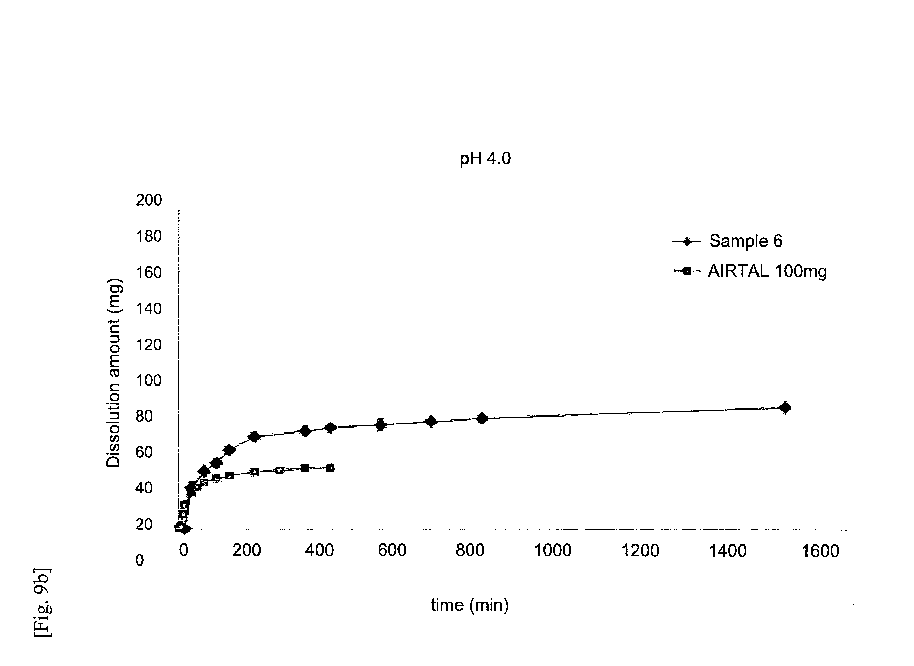

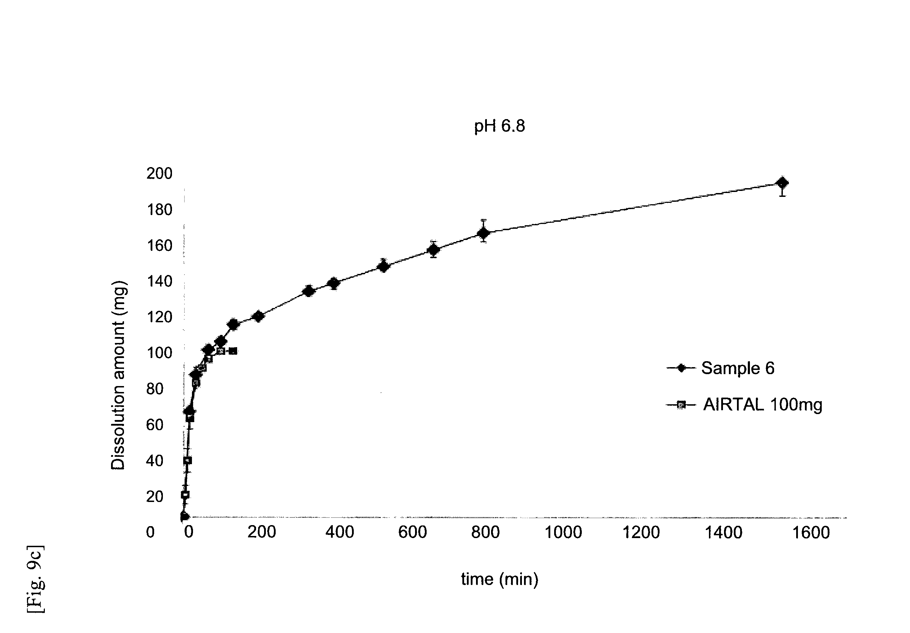

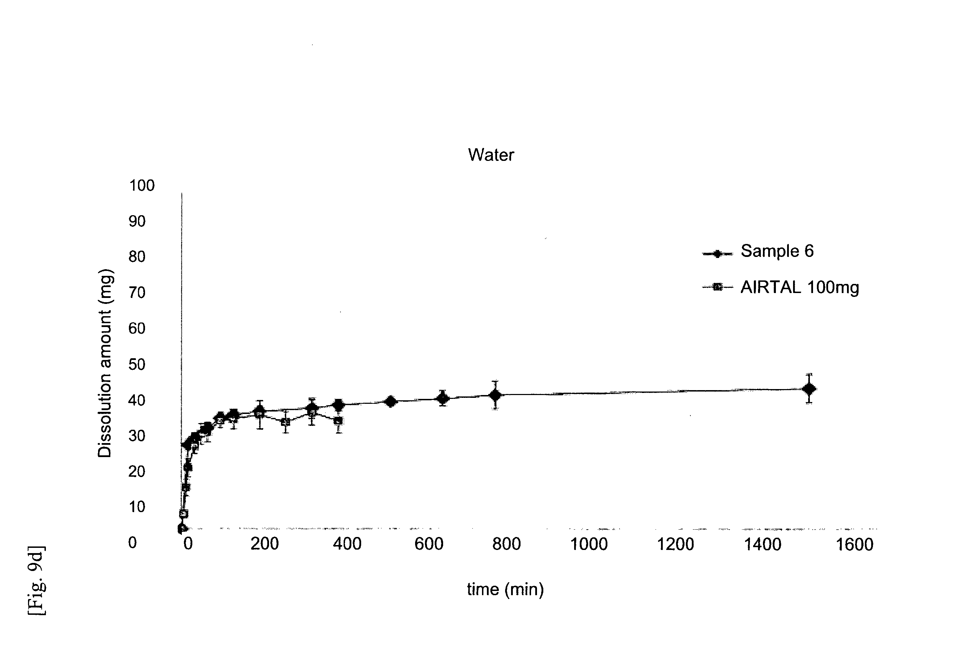

FIGS. 9A to 9D are graphs showing results of a dissolution rate test for an aceclofenac slow-release preparation depending on pH shown in Table 10 according to the present invention;

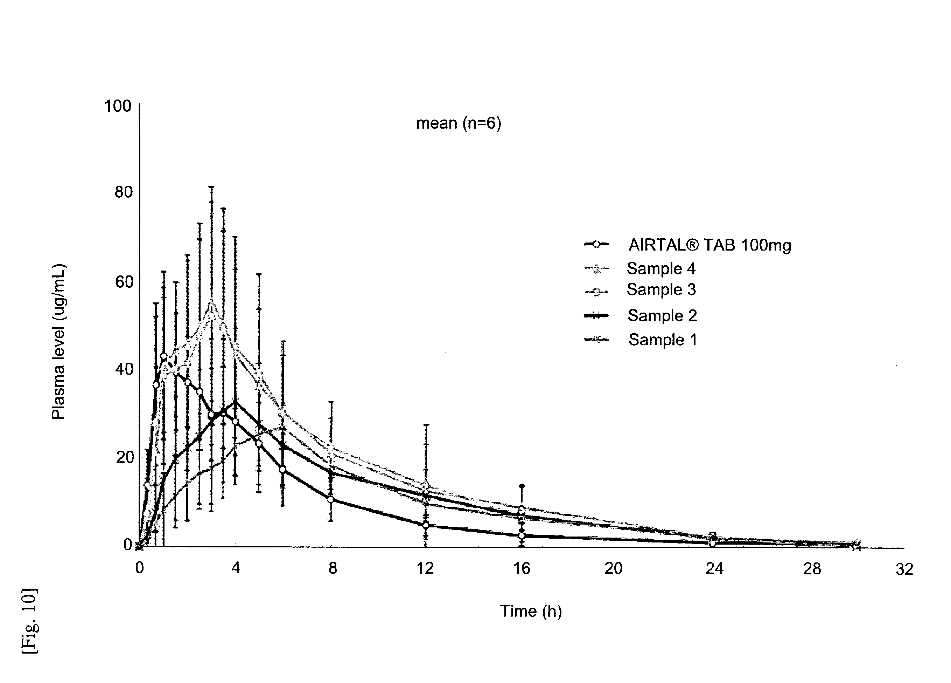

FIG. 10 is a graph showing results of a pre-clinical test for sample preparations 4, 5, 6 and 7 and Airtal^{™} (Daewoong Pharmaceutical co., Ltd.), which is commercially available, depending on a weight of a solubilizer shown in Table 10 obtained by measuring a pharmacokinetic coefficient according to the present invention; and

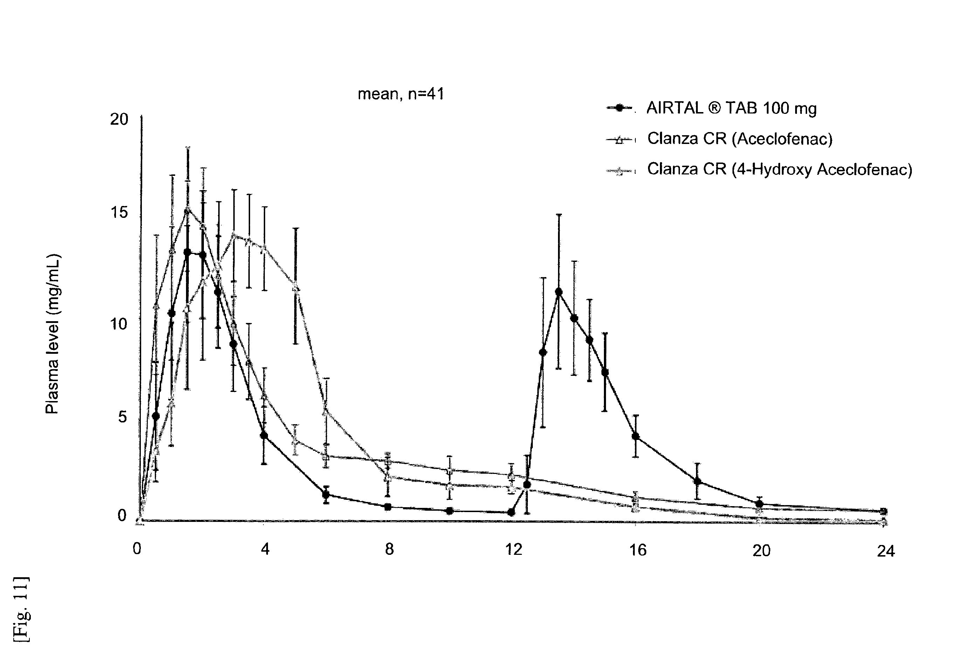

FIG. 11 is a graph showing results of a clinical test for sample preparation 7 and Airtal^{™} (Daewoong Pharmaceutical Co., Ltd.), which is commercially available, obtained by measuring a pharmacokinetic coefficient according to the present invention.

### [Mode for Invention]

Hereinafter, the composition of the present invention will be described in detail.

In the present invention, the term "aceclofenac" includes all kinds of compounds including a pharmaceutically available aceclofenac salt, diclofenac, 9 kinds of metabolites (including 4-hydroxyaceclofenac, 4-hydroxydiclofenac, 5-hydroxyaceclofenac, 5-hydroxydiclofenac, etc.), and derivatives thereof that are capable of being metabolized into diclofenac in a living body.

A content of aceclofenac contained in an oral preparation may be 200 mg, a content of aceclofenac contained in an immediate-release layer may be 40 to 100 mg, and preferably 70 to 90 mg, and a content of aceclofenac contained in a slow-release layer may be 100 to 160 mg, and preferably 110 to 130 mg. Here, the sum of the contents of aceclofenac contained in the immediate-release layer and the slow-release layer may be 300 to 800 mg, and most preferably, 450 to 470 mg, so as to satisfy a high initial blood concentration and consistently maintain an effective blood concentration as well. In addition, due to the contents described above, the oral preparation can be administered once a day.

A controlled-release aceclofenac preparation of the present invention also includes any mixtures and composites of metabolites and aceclofenac derivatives as well as a preparation including an immediate-release layer and a slow-release layer, and a method of preparing a bilayered, dual or multilayered oral preparation to provide a pharmaceutical composition is provided.

In detail, the oral preparation may be prepared using a complex tablet press, a multilayered tablet press or a dual tablet press, by preparing wet and dry slow-release granules by mixing aceclofenac, a polymer base for release control and an additive, and mixing a lubricant with a mixture containing each of the slow-release granules and aceclofenac.

As a representative example of the present invention, a bilayered tablet composed of an immediate-release layer and a slow-release layer may be prepared in the form of a multilayered tablet by primarily compressing a slow-release layer into a tablet, filling a formulation for an immediate-release layer thereon, and secondarily compressing an immediate-release layer into a tablet. Here, the compressing of an immediate-release layer is not necessarily performed after the compressing of a slow-release layer. Therefore, after the compressing of an immediate-release layer into a tablet is primarily performed, granules for forming a slow-release layer are filled, and the compressing of a slow-release layer into a tablet may be performed. Alternatively, the immediate-release layer and the slow-release layer may be filled sequentially or *vice versa* and then compressed once so as to form a tablet.

In addition, the tablet of the present invention may be formed in a triple-layered tablet composed of an immediate-release layer and a slow-release layer, or a monolayered tablet including a mixed composition of a composition for an immediate-release layer and a composition for a slow-release layer and simultaneously having immediate-release characteristics and slow-release characteristics. Alternatively, the tablet may be prepared in a multi tablet including a slow-release core by forming a slow-release layer as a core and surrounding the slow-release layer with an immediate-release layer.

The base for release control used in the oral preparation of the present invention includes a "pH-dependent polymer base" and a "pH-independent polymer base." The "pH-independent polymer base" refers to a substance for controlling and delaying the release of a drug by absorbing moisture regardless of pH change, and the "pH-dependent polymer base" refers to a substance for controlling and delaying the release of a drug by absorbing moisture in sensitive response to pH change.

The polymer for release control may be any one of the pharmaceutically available polymers, which may include one or a mixture of at least two selected from the group consisting of cellulose derivatives composed of hydroxypropylmethyl cellulose, methyl cellulose, ethyl cellulose, hydroxy methylcellulose and sodium carboxymethylcellulose, propylene oxide and a derivative thereof, polyvinylpyrrolidone (molecular weight: 90, product name: Povidone K-90), polyethylene glycol, polyvinyl alcohols, polyvinylacetate, polyvinylacetate phthalate, polymethacrylate, a polymer of polymethacrylate (commercially available as "Eudragit"), polyacrylic acid, a derivative of polymethacrylate (e.g., a carbomer), glycerolmonostearate, and poloxamer. Preferably, the polymer for release control includes one or a mixture of at least two selected from the group consisting of HPMC, a carbomer, hydroxypropylcellulose, methylcellulose, polyvinylpyrrolidone and polyvinylalcohol.

When a general polymer for release control is used, a dissolution rate of the drug is not uniform, and the polymer is not suitable for a slow-release tablet since most effective components are released in the beginning of administration. To solve these problems, HPMC and a carbomer are used as a polymer for release control and an auxiliary polymer for release control in the present invention.

As HPMC, many products having various viscosities are known, and the dissolution rate of the slow-release aceclofenac tablet may be regulated by regulating the viscosity of HPMC. HPMC included in the slow-release aceclofenac tablet according to the present invention may be HPMC having high viscosity, which may be 60,000 to 140,000 cps, and preferably 80,000 to 120,000 cps. When the viscosity is less than 60,000 cps, the tablet becomes larger in size, and when the viscosity is more than 140,000 cps, HPMC is difficult to uniformly mix with the drug.

A slow-release tablet containing a pharmaceutically effective component is swollen when being dissolved. In this case, if a matrix of the polymer for release control is not firm, a part of the matrix may be eroded, thereby disintegrating the tablet, which leads to rapid release of the drug. Therefore, the rapid release of the drug may cause a patient to have headaches or flush. For this problem, in the present invention, as a polymer for release control, a mixture of HPMC and a carbomer was used. When the carbomer is used with HPMC as a polymer for controlling release of a drug, it has an effect of firming the matrix in the slow-release tablet and maintaining a shape of the tablet and the matrix of the tablet when the tablet is swollen. Therefore, the erosion of the tablet is prevented, and the dissolution rate is uniformly maintained.

To prepare the slow-release aceclofenac tablet which can be administered once a day, a weight ratio of HPMC and the carbomer may be 1:1 to 30:1. When the weight ratio is less than 1:1, it is difficult to form a matrix in the tablet, thereby degrading the effect of delaying drug release, whereas, when the weight ratio is more than 30:1, the dissolution rate of the drug is decreased under an alkali condition, thereby causing difficulty in uniform mixing between polymers for release control.

In an exemplary embodiment of the present invention, an amount of HPMC used as a pH-independent polymer base may be 10 to 60 parts by weight, and preferably 40 to 55 parts by weight, based on a total weight of the slow-release layer.

In addition, in the pH-dependent polymer base used in the oral preparation of the present invention, when a system dissolved under an acidic condition (in the stomach) is operated in a pH-dependent manner, a hydrogel is formed to surround the drug, and thus the drug release is controlled. The pH-dependent polymer base used in the preparation of the present invention may be a water-soluble salt of alginic acid such as sodium alginate, polyacrylic acids and derivatives thereof (e.g., carbomers), and preferably a carboxyvinyl polymer.

In an exemplary embodiment of the present invention, an amount of the pH-dependent polymer used herein may be 2 to 10 parts by weight, and preferably 2 to 6 parts by weight based on a total weight of the slow-release layer.

It is known that the HPMC and carbomers are used for various pharmaceutical purposes according to the molecular weights thereof [Handbook of Pharmaceutical Excipients, Edited by Raymond C Rowe, Paul J sheskey and Paul weller, 2003, fourth edition, APhA].

In addition, a disintegrating agent used in the oral preparation of the present invention is used to absorb moisture, stimulate disintegration of the preparation, and improve dissolution of aceclofenac. The disintegrating agent capable of being used in the preparation of the present invention may include one or a mixture selected from the group consisting of croscarmellose sodium, sodium starch glycolate, pregelatinized starch (Starch 1500 or Prejel), microcrystalline cellulose, crospovidone, cross-linked povidone and other commercially useful polyvinylpyrrolidone (PVP, povidone), low-substituted hydroxypropylcellulose, alginic acid, calcium and nitrogen salts of carboxymethylcellulose, colloidal silicon dioxide (fumed silica and colloidal silica), guar gum, magnesium aluminum silicate, methylcellulose, powdery cellulose, starch and sodium alginate.

Preferably, the disintegrating agent is croscarmellose sodium, sodium starch glycolate, pregelatinized starch, microcrystalline cellulose, crospovidone or commercially available polyvinylpyrrolidone. More preferably, the disintegrating agent is crospovidone, sodium starch glycolate or microcrystalline cellulose, and particularly a mixture of at least two thereof is the most effective disintegrating agent. The disintegrating agent may be further added to a solid oral preparation by a pharmaceutically available method, and a secondary disintegrating agent may be further used for a purpose of more rapid release of the preparation. The disintegrating agent may be used in an amount of 4.5 to 10 parts by weight, and preferably 6 to 8 parts by weight based on a total weight thereof.

In addition, a lubricant used in the oral preparation of the present invention enhances plasticity of the oral preparation, and may be, but is not limited to, magnesium stearate, silicon oxide (SiO₂), colloidal silica (Cabo-SIL) or talc. An amount of the lubricant may be used at 6 to 20 parts by weight based on a total weight of the oral preparation.

The slow-release oral reparation of the present invention may include a pharmaceutically commercially available additive, which may be lactose, sugar, mannitol, lactose or sorbitol, and further include a preserve or stabilizer when necessary.

A solvent added to a powder mixture in preparation of wet granules of the present invention may be one or a mixture selected from the group consisting of water, ethanol, isopropylalcohol, glycerin, propyleneglycol and polyethyleneglycol, and preferably ethanol or a mixture of water and ethanol.

A solubilizer used in the oral preparation of the present invention was used to increase bioavailability in the gastric juice by enhancing solubility and stability of insoluble and unstable aceclofenac under an acidic condition.

Here, the solubilizer may be, but is not limited to, poloxamer, an organic acid and an organic acid salt monoglyceride derivative, glyceride and a derivative thereof, diethylene glycol ester, sorbitan ester, carbonate salt and polyoxyethylene ether, Gelucire 44/14, polyethylene glycols, Brijs, Labrafils and sodium laurylsulfate, all of which are pharmaceutically available.

The aceclofenac oral preparation according to the prior art showed different dissolution profiles depending on pH when the dissolution of the drug was compared in water and dissolving solutions having pH of 1.2, 4.0 and 6.8. Moreover, it can be confirmed that, under an acidic condition, a softening substance of aceclofenac is generated. Therefore, to solve this problem, a stabilizer was used with the solubilizer, thereby confirming suitable solubility and stability under an acidic condition (the gastric juice having pH 1.2), and showing uniform dissolution profiles and stability even when pH increases.

As the dissolution profile and pharmacological behavior of the oral preparation were compared, pharmacological activities of the composite preparation of the exemplary embodiment were confirmed in beagle dogs so as to predict an effect of an increase in solubility and enhancement of stability of aceclofenac on expression of drug effects in humans. As a result of the experiment, it could be confirmed that the pharmacological behavior of aceclofenac was changed according to a dissolution profile *in vitro,* and a suitable dissolution profile of aceclofenac *in vitro* leads to suitable pharmacological activities of aceclofenac *in vivo.*

A bilayered tablet composed of an immediate-release outlet and a slow-release outlet, both of which include the same active components, was eluted by a paddle method (the Korean Pharmacopoeia, 9th ed., Second Method of Dissolution Test) of 50 cycles per minute using 900 ml of artificial intestinal juice (the Korean Pharmacopoeia, 9th ed., Second liquid for a disintegration test, pH 6.8) as a dissolving solution, and dissolution rates within 1, 12 and 24 hours after initiation of the dissolution test were 45 to 65%, 65 to 85%, and 85% or more, respectively.

Hereinafter, the present invention will be described in detail with reference to Examples. However, the technical idea of the present invention is not limited to the following Examples.

### [Experimental Example 1]

To confirm the interaction and compatibility between aceclofenac and an excipient, a test was performed as follows:

After manually mixing 200 mg of aceclofenac and each of the excipients, an acceleration test was performed at room temperature for 1 hour, the mixture was left in an atmosphere having a humidity of 75% for 1 month, and a content test and a softening substance test were performed. Test results are shown in Table 1.

**[Table 1]**

| Excipient | Early Content Test | | | Test for Content After Acceleration Test | | |
|---|---|---|---|---|---|---|
| | Reference (%) | Result (%) | Determination | Reference (%) | Result (%) | Determination |
| Microcrystalline cellulose | 95-105 | 99.5 | suitable | 95-105 | 99.3 | suitable |
| Lactose monohydrate | 95-105 | 99.1 | suitable | 95-105 | 99.2 | suitable |
| Hydroxypropyl cellulose | 95-105 | 99.3 | suitable | 95-105 | 99.2 | suitable |
| Hydromellose phthalate | 95-105 | 99.9 | suitable | 95-105 | 99.2 | suitable |
| Carboxymethylcellulose sodium | 95-105 | 99.9 | suitable | 95-105 | 100 | suitable |
| Carboxymethylcellulose calcium | 95-105 | 99.8 | suitable | 95-105 | 99.8 | suitable |
| Copovidone | 95-105 | 99.3 | suitable | 95-105 | 99.3 | suitable |
| Aerosil | 95-105 | 99.5 | suitable | 95-105 | 99.4 | suitable |
| Poloxamer | 95-105 | 99.4 | suitable | 95-105 | 99.4 | suitable |
| PVP K-30 | 95-105 | 99.3 | suitable | 95-105 | 100 | suitable |
| HPMC 2208 (100,000 cp) | 95-105 | 99.8 | suitable | 95-105 | 99.8 | |
| HPMC2910(4000 cp) | 95-105 | 99.1 | suitable | 95-105 | 99.4 | suitable |
| Carbopol 971p NF | 95-105 | 99.7 | suitable | 95-105 | 99.3 | suitable |
| Crospovidone | 95-105 | 99.7 | suitable | 95-105 | 99.2 | suitable |
| Magnesium stearate | 95-105 | 99.6 | suitable | 95-105 | 99.3 | suitable |
| Croscarmellose sodium | 95-105 | 99.5 | suitable | 95-105 | 99.6 | suitable |
| Sodium starch (pregelatinized) | 95-105 | 99.4 | suitable | 95-105 | 100 | suitable |
| Hydroxypropyl cellulose low-substituted | 95-105 | 99.6 | suitable | 95-105 | 99.5 | suitable |
| Mannitol | 95-105 | 99.5 | suitable | 95-105 | 99.6 | suitable |
| Polyethylene glycol 6000 | 95-105 | 99.1 | suitable | 95-105 | 99.6 | suitable |
| Sucrose | 95-105 | 99.5 | suitable | 95-105 | 99.5 | suitable |
| Polyvinyl alcohol | 95-105 | 99.5 | suitable | 95-105 | 100 | suitable |
| Hydroxyethyl cellulose | 95-105 | 99.6 | suitable | 95-105 | 100.3 | suitable |
| Sodium alginate | 95-105 | 99.5 | suitable | 95-105 | 99.8 | suitable |

### [Experimental Example 2]

The degree of solubilization of aceclofenac was measured by the following test method.

20 mg of aceclofenac was dispersed in 40 ml of water, and then dissolved by addition of 10 mg of a solubilizer. An aceclofenac suspension was left at room temperature for 24 hours, and filtered using a 0.45 µm membrane filter. Then, the filtrate was run through HPLC to confirm the solubility and softening substance of aceclofenac. The results are shown in Table 2.
UV detector: Jasco UV-975
Wavelength: 282 nm
Column: Haisil ODS 15 cm * 4.6 mm
Mobile phase: -65% MeOH: 35% 0.02 M potassium dihydrogenphosphate
Flow rate: 1.0 ml/min
Injection volume: 20 µl

**[Table 2]**

| Surfactant | Solubility | Surfactant | Solubility |
|---|---|---|---|
| Cremophor EL | 0.117±0.0012 | Tween 80 | 0.282±0.0040 |
| Cremophor RH 40 | 0 | Glycerin | 0.187±0.0003 |
| Gelucire 44/14 | 0.465±0.0008 | Brij 98 | 0.118±0.0005 |
| Rabrafac cc | 0.165±0.0003 | Aracel 83 | 1.377±0.0007 |
| Polyethyleneglycol 300 | 0.327±0.0024 | Brij 35 | 1.630±0.0015 |
| Polyethyleneglycol 400 | 0.209±0.0007 | Brij 56 | 1.090±0.0013 |
| Polyethyleneglycol 600 | 0.218±0.0023 | Brij 58 | 1.207±0.0031 |
| Polyethyleneglycol 6000 | 0.529±0.00311 | Brij 92 | 0.645±0.0002 |
| Poloxamer 407 | 1.854±0.0007 | Brij 92 | 1.418±0.0006 |
| Povidone k-30 | not measure | Labrafil | 0.846±0.0009 |
| Tefose 63 | 0.183±0.0009 | Span 2ß | 0.294±0.0027 |
| Tefose 70 | 0.524±0.0016 | Span 40 | 0.379±0.0016 |
| Transcutol | 0.169±0.0094 | Span 60 | 0.628±0.0008 |
| Tween 20 | 0.724±0.0041 | Span 80 | 0.424t0.0011 |
| Tween 60 | 0.628±0.0035 | SLS | 1.633±0.0024 |

| | | | |
|---|---|---|---|
| The aceclofenac was added into each excipient solution (mg/ml (n=3, mean ±S.D). | | | |

The results for confirming the softening substance of aceclofenac are shown in Table 3.

**[Table 3]**

| Surfactant type | 1 day | 2 day | 3 day |
|---|---|---|---|
| Cremophor EL | 0.695 ±0.0008 | 1.494 ±0.0007 | 2.392 ±0.0002 |
| Cremophor RH 40 | 0.942 ±0.0005 | 2.365 ±0.0004 | 3.326 ±0.0009 |
| Gelucire 44/14 | 0.237 ±0.0002 | 0.267 ±0.0003 | 0.276 ±0.0005 |
| Rabrafac cc | 0.061±0.0004 | 0.069 ±0.0001 | 0.239 ±0.0001 |
| Polyethyleneglycol 300 | 0.332 ±0.0008 | 0.490 ±0.0006 | 1.052 ±0.0004 |
| Polyethyleneglycol 400 | 0.218 ±0.0007 | 0.239 ±0.0003 | 0.647 ±0.0008 |
| Polyethyleneglycol 600 | 0.186 ±0.0003 | 0.304 ±0.0004 | 0.548 ±0.0005 |
| Polyethyleneglycol 6000 | 0.478 ±0.0001 | 0.990 ±0.0005 | 1.378 ±0.0001 |
| Poloxamer 407 | 0.466 ±0.0005 | 0.696 ±0.0002 | 1.479 ±0.0004 |
| Povidone k-30 | 0.452 ±0.0005 | 1.121 ±0.0004 | 1.859 ±0.0008 |
| Tefose 63 | 0.231 ±0.0008 | 0.458 ±0.0007 | 0.854 ±0.0005 |
| Tefose 70 | 0.403 ±0.0001 | 0.348 ±0.0008 | 0.622 ±0.0001 |
| Transcutol | 0.297 ±0.0006 | 0.323 ±0.0007 | 0.378±0.0006 |
| Tween 20 | 0.631 ±0.0007 | 1.568 ±0.0004 | 2.042 ±0.0005 |
| Tween 60 | 0.829 ±0.0004 | 1.529 ±0.0003 | 2.241 ±0.0009 |
| Tween 80 | 1.179 ±0.0005 | 1.319 ±0.0003 | 2.162 ±0.0008 |
| Glycerin | 0.018 ±0.0001 | 0.045 ±0.0001 | 0.189 ±0.0004 |
| Brij 98 | 0.325 ±0.0004 | 0.984 ±0.0006 | 1.341 ±0.0007 |
| Aracel 83 | 1.615 ±0.0008 | 1.772 ±0.0001 | 2.059 ±0.0001 |
| Brij 35 | 0.573 ±0.0005 | 1.148 ±0.0002 | 1.308 ±0.0009 |
| Brij 56 | 1.092 ±0.0006 | 1.813 ±0.0008 | 2.787 ±0.0003 |
| Brij 58 | 0.748 ±0.0009 | 1.132 ±0.0008 | 1.560 ±0.0006 |
| Brij 92 | 0.234 ±0.0005 | 0.219 ±0.0007 | 0.856 ±0.0005 |
| Brij 97 | 0.234 ±0.0006 | 1.820 ±0.0003 | 3.080 ±0.0008 |
| Labrafil | 1.011 ±0.0001 | 1.473 ±0.0002 | 1.537 ±0.0003 |
| SLS | 1.345 ±0.0005 | 1.515 ±0.0004 | 1.806 ±0.0008 |
| Span 20 | 0.168 ±0.0003 | 0.178 ±0.0003 | 0.259 ±0.0007 |
| Span 40 | 0.291 ±0.0006 | 0.256 ±0.0001 | 0.334 ±0.0004 |
| Span 60 | 0.474 ±0.0002 | 0.788 ±0.0006 | 0.865 ±0.0004 |
| Span 80 | 0.121 ±0.0008 | 0.149 ±0.0001 | 0.233 ±0.0004 |

| | | | |
|---|---|---|---|
| The % of the related substance was determined based on aceclofenac contents. | | | |

### [Experimental Example 3]

A formation of an immediate-release layer part was determined by measuring physical properties between aceclofenac and each excipient.

### -Test Method

A suitable excipient was selected by performing flowability, hardness and friability tests with respect to a mixture prepared by mixing aceclofenac with each excipient, and then with a lubricant. The test results are shown in Table 4.

**[Table 4]**

| Formulation | | Friability Test | Hardness Test | Flowability Test | Disintegration Test | Dissolution Test |
|---|---|---|---|---|---|---|
| F1 | Aceclofenac (100 mg) Flowlac (80 mg) Magnesium stearate (4 mg) | Not suitable | Suitable | - | - | - |
| F2 | Aceclofenac (100 mg) Flowlac (100 mg) Magnesium stearate (4 mg) | Not suitable | Suitable | - | - | - |
| F3 | Aceclofenac (100 mg) Flowlac (120 mg) Magnesium stearate (4 mg) | Not suitable | Suitable | - | - | - |
| F4 | Aceclofenac (100 mg) D-mannitol (80 mg) Magnesium stearate (4 mg) | Not suitable | Suitable | - | - | - |
| F5 | Aceclofenac (100 mg) D-mannitol (100 mg) Magnesium stearate (4 mg) | Not suitable | Suitable | - | - | - |
| F6 | Aceclofenac (100 mg) D-mannitol (120 mg) Magnesium stearate (4 mg) | Not suitable | Suitable | - | - | - |
| F7 | Aceclofenac (100 mg) Flowlac (100 mg) Corn starch (10 mg) Magnesium stearate (4 mg) | Suitable | Suitable | Not suitable | - | - |
| F8 | Aceclofenac (100 mg) Flowlac (100 mg) Corn starch (20 mg) Magnesium stearate (4 mg) | Suitable | Suitable | Not suitable | - | - |
| F9 | Aceclofenac (100 mg) Flowlac (100 mg) Corn starch (30 mg) Magnesium stearate (4 mg) | Suitable | Suitable | Not suitable | - | - |
| F10 | Aceclofenac (100 mg) Flowlac ( 100 mg) MCC pH102 (10 mg) Magnesium stearate (4 mg) | Suitable | Suitable | Not suitable | - | - |
| F11 | Aceclofenac (100 mg) Flowlac (100 mg) MCC pH102 (20 mg) Magnesium stearate (4 mg) | Suitable | Suitable | Not suitable | - | - |
| F12 | Aceclofenac (100 mg) Flowlac (100 mg) MCC pH102 (30 mg) Magnesium stearate (4 mg) | Suitable | Suitable | Not suitable | - | - |
| F13 | Aceclofenac (100 mg) Flowlac (100 mg) MCC pH102 (10 mg) Aerosil 200 (5 mg) Magnesium stearate (4 mg) | Suitable | Suitable | Suitable | Not suitable | - |
| F14 | Aceclofenac (100 mg) Flowlac (100 mg) MCC pH 102 (10 mg) Aerosil 200 (10 mg) Magnesium stearate (4 mg) | Suitable | Suitable | Suitable | Not suitable | - |
| F15 | Aceclofenac (100 mg) Flowlac (100 mg) MCC pH 102 (10 mg) Aerosil 200 (15 mg) Magnesium stearate (4 mg) | Suitable | Suitable | Suitable | Not suitable | - |
| F16 | Aceclofenac (100 mg) Flowlac (100 mg) MCC pH 102 (10 mg) Aerosil 300 (5 mg) Magnesium stearate (4 mg) | Suitable | Suitable | Suitable | Not suitable | - |
| F17 | Aceclofenac (100 mg) Flowlac (100 mg) MCC pH 102 (10 mg) Aerosil 300 (10 mg) Magnesium stearate (4 mg) | Suitable | Suitable | Suitable | Not suitable | - |
| F18 | Aceclofenac (100 mg) Flowlac (100 mg) MCC pH 102 (10 mg) Aerosil 300 (15 mg) Magnesium stearate (4 mg) | Suitable | Suitable | Suitable | Not suitable | - |
| F19 | Aceclofenac (100 mg) Flowlac (100 mg) MCC pH 102 (10 mg) SLS (5 mg) Magnesium stearate (4 mg) | Suitable | Suitable | Suitable | Not suitable | |
| F20 | Aceclofenac (100 mg) Flowlac (100 mg) MCC pH 102 (10 mg) SLS (10 mg) Magnesium stearate (4 mg) | Suitable | Suitable | Suitable | Not suitable | - |
| F21 | Aceclofenac (100 mg) Flowlac (100 mg) MCC pH102 (10 mg) SLS (15 mg) Magnesium stearate (4 mg) | Suitable | Suitable | Suitable | Not suitable | - |
| F22 | Aceclofenac (100 mg) Flowlac (100 mg) MCC pH102 (10 mg) Talc (5 mg) Magnesium stearate (4 mg) | Suitable | Suitable | Suitable | Not suitable | - |
| F23 | Aceclofenac (100 mg) Flowlac (100 mg) MCC pH102 (10 mg) Talc (10 mg) Magnesium stearate (4 mg) | Suitable | Suitable | Suitable | Not suitable | - |
| F24 | Aceclofenac (100 mg) Flowlac (100 mg) MCC pH102 (10 mg) Talc (15 mg) Magnesium stearate (4 mg) | Suitable | Suitable | Suitable | Not suitable | - |
| F25 | Aceclofenac (100 mg) Flowlac (100 mg) MCC pH102 (10 mg) Aerosil 300 (5 mg) CL-PVP (5 mg) Magnesium stearate (4 mg) | Suitable | Suitable | Suitable | Suitable | Not suitable |
| F26 | Aceclofenac (100 mg) Flowlac (100 mg) MCC pH102 (10 mg) Aerosil 300 (5 mg) CL-PVP (10 mg) Magnesium stearate (4 mg) | Suitable | Suitable | Suitable | Suitable | Not suitable |
| F27 | Aceclofenac (100 mg) Flowlac (100 mg) MCC pH102 (10 mg) Aerosil 300 (5 mg) CL-PVP (15 mg) Magnesium stearate (4 mg) | Suitable | Suitable | Suitable | Suitable | Not suitable |
| F28 | Aceclofenac (100 mg) Flowlac (100 mg) MCC pH102 (10 mg) Aerosil 300 (5 mg) CL-PVP (20 mg) Magnesium stearate (4 mg) | Suitable | Suitable | Suitable | Suitable | Not suitable |
| F29 | Aceclofenac (100 mg) Flowlac (100 mg) MCC pH102 (10 mg) Aerosil 300 (5 mg) Croscarmellose sodium (5 mg) Magnesium stearate (4 mg) | Suitable | Suitable | Suitable | Suitable | Not suitable |
| F30 | Aceclofenac (100 mg) Flowlac (100 mg) MCC pH 102 (10 mg) Aerosil 300 (5 mg) Croscarmellose sodium (10 mg) Magnesium stearate (4 mg) | Suitable | Suitable | Suitable | Suitable | Not suitable |
| F31 | Aceclofenac (100 mg) Flowlac (100 mg) MCC pH102 (10 mg) Aerosil 300 (5 mg) Croscarmellose sodium (15 mg) Magnesium stearate (4 mg) | Suitable | Suitable | Suitable | Suitable | Not suitable |
| F32 | Aceclofenac (100 mg) Flowlac (100 mg) MCC pH102 (10 mg) Aerosil 300 (5 mg) Croscarmellose sodium (20 mg) Magnesium stearate (4 mg) | Suitable | Suitable | Suitable | Suitable | Not suitable |
| F33 | Aceclofenac (100 mg) Flowlac (100 mg) MCC pH 102 (10 mg) Aerosil 300 (5 mg) Starch 1500 (5 mg) Magnesium stearate (4 mg) | Suitable | Suitable | Suitable | Suitable | Not suitable |
| F34 | Aceclofenac (100 mg) Flowlac (100 mg) MCC pH102 (10 mg) Aerosil 300 (5 mg) Starch 1500 (10 mg) Magnesium stearate (4 mg) | Suitable | Suitable | Suitable | Suitable | Not suitable |
| F35 | Aceclofenac (100 mg) Flowlac (100 mg) MCC pH 102 (10 mg) Aerosil 300 (5 mg) Starch 1500 (15 mg) Magnesium stearate (4 mg) | Suitable | Suitable | Suitable | Suitable | Not suitable |
| F36 | Aceclofenac (100 mg) Flowlac (100 mg) MCC pH102 (10 mg) Aerosil 300 (5 mg) Starch 1500 (20 mg) Magnesium stearate (4 mg) | Suitable | Suitable | Suitable | Suitable | Not suitable |
| F37 | Aceclofenac (100 mg) Flowlac (100 mg) MCC pH102 (10 mg) Aerosil 300 (5 mg) CL-PVP (5 mg) Poloxamer (5 mg) Magnesium stearate (4 mg) | Suitable | Suitable | Suitable | Suitable | Not suitable |
| F38 | Aceclofenac (100 mg) Flowlac (100 mg) MCC pH102 (10 mg) Aerosil 300 (5 mg) CL-PVP (10 mg) Poloxamer (7 mg) Magnesium stearate (4 mg) | Suitable | Suitable | Suitable | Suitable | Suitable |
| F39 | Aceclofenac (100 mg) Flowlac (100 mg) MCC pH102 (10 mg) Aerosil 300 (5 mg) CL-PVP (10 mg) Poloxamer (3 mg) Magnesium stearate (4 mg) | Suitable | Suitable | Suitable | Suitable | Not suitable |
| F40 | Aceclofenac (100 mg) Flowlac (100 mg) MCC pH102 (10 mg) Aerosil 300 (5 mg) Gelucire 44/14 (5 mg) Magnesium stearate (4 mg) | Suitable | Suitable | Suitable | Suitable | Not suitable |
| F41 | Aceclofenac (100 mg) Flowlac (100 mg) MCC pH102 (10 mg) Aerosil 300 (5 mg) Gelucire 44/14 (7 mg) Magnesium stearate (4 mg) | Suitable | Suitable | Suitable | Suitable | Not suitable |
| F42 | Aceclofenac (100 mg) Flowlac (100 mg) MCC pH102 (10 mg) Aerosil 300 (5 mg) Gelucire 44/14 (10 mg) Magnesium stearate (4 mg) | Suitable | Suitable | Suitable | Suitable | Not suitable |
| F43 | Aceclofenac (100 mg) Flowlac (100 mg) MCC pH102 (10 mg) Aerosil 300 (5 mg) CL-PVP (5 mg) Poloxamer (3 mg) Magnesium stearate (4 mg) | Suitable | Suitable | Suitable | Suitable | Suitable |
| F44 | Aceclofenac (100 mg) Flowlac (100 mg) MCC pH102 (10 mg) Aerosil 300 (5 mg) CL-PVP (10 mg) Poloxamer (8 mg) Magnesium stearate (4 mg) | Suitable | Suitable | Suitable | Suitable | Suitable |
| F45 | Aceclofenac (100 mg) Flowlac (100 mg) MCC pH102 (10 mg) Aerosil 300 (5 mg) CL-PVP (10 mg) Poloxamer (5 mg) Magnesium stearate (4 mg) | Suitable | Suitable | Suitable | Suitable | Suitable |

According to the test results, crosspovidone and poloxamer are the most suitable for a disintegrating agent and a solubilizer, respectively, in terms of the physical properties, and a ratio of the two components is most preferably 1.2:1 to 1.7:1. It can be noted that a formulation is not suitable in terms of the dissolution rate when the two components are included beyond the above-mentioned ratio.

### [Experimental Example 4]

A formulation of a slow-release layer part is determined by measuring physical properties between aceclofenac and each excipient in the formulation of the immediate-release layer determined in Experimental Example 3.

### -Method of Preparing Slow-Release Layer Granule

After 100.0 mg of a drug, aceclofenac and each excipient were sufficiently mixed, 85.0 mg of HPMC as a polymer base was added to and uniformly mixed in a powder mixer, and sprayed with ethanol, thereby preparing wet granules.

10 ml of ethanol was used to prepare 100 typical tablets. When necessary, a small amount of the polymer base of the formulation may be dissolved in a solvent mixture of water or alcohol, and then used to granulize powder.

The prepared granules were sufficiently dried in an oven at 60 °C and evenly milled, and then magnesium stearate was added thereto to plasticize the preparation. The mixed granules of the immediate-release layer were compressed using a bilayered tablet rotary tableting machine into a bilayered tablet containing aceclofenac.

A formulation of the slow-release layer part of the preparation was determined with F38 of the immediate-release layer part determined in Table 4. According to the test results, the flowability of the granules during the preparation of the bilayered tablet and the hardness of the tablet were increased depending on a content of lactose or microcrystalline cellulose, and to have a dissolution rate lasting for 24 hours, components of HPMC 2280 and 2910 were used together in a ratio of approximately 4:1 as a pH-independent polymer base. Referring to the test results, it could be confirmed that a dissolution profile of aceclofenac from the slow-release part was dependent on a ratio of the contents of a slow-releasing base, HPMC, and a carbomer. Therefore, the dissolution profile according to the ratio of additional HPMC and carbomer was confirmed as will be described in the following Experimental Example 5. According to the following test, it seems that F28, F29 and F30 are preferable.

**[Table 5]**

| Formulation | | | Hardness Test | Flowability Test | Dissolution Test |
|---|---|---|---|---|---|
| | Immediate-Release Layer | Slow-Release Layer | | | |
| F1 | Aceclofenac (100 mg) | Aceclofenac (100 mg) | Suitable | Not suitable | - |
| | | Kolidone SR (20 mg) | | | |
| | Flowlac (100 mg) | Magnesium stearate (4 mg) | | | |
| | MCC pH102 (10 mg) | | | | |
| F2 | Aerosil 300 (5 mg) | Aceclofenac (100 mg) | Suitable | Not suitable | - |
| | CL-PVP (10 mg) | Kolidone SR (30 mg) | | | |
| | Poloxamer (7 mg) | Magnesium stearate (4 mg) | | | |
| F3 | Magnesium stearate (4 mg) | Aceclofenac (100 mg) | Suitable | Not suitable | - |
| | | Kolidone SR (40 mg) | | | |
| | | Magnesium stearate (4 mg) | | | |
| F4 | Aceclofenac (100 mg) | Aceclofenac (100 mg) | Suitable | Not suitable | - |
| | | HPMC 2208 (100,000 cps, 20 mg) | | | |
| | Flowlac (100 mg) | | | | |
| | MCC pH 102(10 mg) | | | | |
| | | Magnesium stearate (4 mg) | | | |
| | Aerosil 300 (5 mg) | | | | |
| F5 | CL-PVP (10 mg) | Aceclofenac (100 mg) | Suitable | Not suitable | - |
| | Poloxamer (7 mg) | HPMC 2208 (100,000 cps, 30 mg) | | | |
| | Magnesium stearate (4 mg) | | | | |
| | | Magnesium stearate (4 mg) | | | |
| F6 | | Aceclofenac (100 mg) | Suitable | Not suitable | - |
| | | HPMC 2208 (100,000 cps, 40 mg) | | | |
| | | Magnesium stearate (4 mg) | | | |
| F7 | Aceclofenac (100 mg) | Aceclofenac (100 mg) | Suitable | Suitable | - |
| | | HPMC 2208 (100,000 cps, 20 mg) | | | |
| | Flowlac (100 mg) | | | | |
| | MCC pH102 (10 mg) | Lactose (50 mg) | | | |
| | | Magnesium stearate (4 mg) | | | |
| | Aerosil 300 (5 mg) | | | | |
| F8 | CL-PVP (10 mg) | Aceclofenac (100 mg) | Suitable | Suitable | Not suitable |
| | Poloxamer (7 mg) | HPMC 2208 (100,000 cps, 20 mg) | | | |
| | Magnesium stearate (4 mg) | | | | |
| | | Lactose (60 mg) | | | |
| | | Magnesium stearate (4 mg) | | | |
| F9 | | Aceclofenac (100 mg) | Suitable | Suitable | Not suitable |
| | | HPMC 2208 (100,000 cps, 20 mg) | | | |
| | | Lactose (70 mg) | | | |
| | | Magnesium stearate (4 mg) | | | |
| F10 | Aceclofenac (100 mg) | Aceclofenac (100 mg) | Suitable | Suitable | Not suitable |
| | | HPMC 2208 (100,000 cps, 20 mg) | | | |
| | Flowlac (100 mg) | | | | |
| | MCC pH102 (10 mg) | MCC pH 101 (50 mg) | | | |
| | | Magnesium stearate (4 mg) | | | |
| | Aerosil 300 (5 mg) | | | | |
| F11 | CL-PVP (10 mg) | Aceclofenac (100 mg) | Suitable | Suitable | Not suitable |
| | Poloxamer (7 mg) | HPMC 2208 (100,000 cps, 20 mg) | | | |
| | Magnesium stearate (4 mg) | | | | |
| | | MCC pH 101 (60 mg) | | | |
| | | Magnesium stearate (4 mg) | | | |
| F12 | | Aceclofenac (100 mg) | Suitable | Suitable | Not suitable |
| | | HPMC 2208 (100,000 cps, 20 mg) | | | |
| | | MCC pH 101 (70 mg) | | | |
| | | Magnesium stearate (4 mg) | | | |
| F13 | Aceclofenac (100 mg) | Aceclofenac (100 mg) | Suitable | Suitable | Not suitable |
| | | HPMC 2208 (100,000 cps, 20 mg) | | | |
| | Flowlac (100 mg) | | | | |
| | MCC pH102 (10 mg) | MCC pH 101 (60 mg) | | | |
| | | Carbomer (3 mg) | | | |
| | Aeroperl 300 (5 mg) | Magnesium stearate (4 mg) | | | |
| F14 | CL-PVP (10 mg) | Aceclofenac (100 mg) | Suitable | Suitable | Not suitable |
| | Poloxamer (7 mg) | HPMC 2208 (100,000 cps, 20 mg) | | | |
| | Magnesium stearate (4 mg) | | | | |
| | | MCC pH 101 (60 mg)\ Carbomer (4 mg) | | | |
| | | Magnesium stearate (4 mg) | | | |
| F15 | | Aceclofenac (100 mg) | Suitable | Suitable | Not suitable |
| | | HPMC 2208 (100,000 cps, 20 mg) | | | |
| | | MCC pH 101 (60 mg) | | | |
| | | Carbomer (5 mg) | | | |
| | | Magnesium stearate (4 mg) | | | |
| F16 | Aceclofenac (100 mg) | Aceclofenac (100 mg) | Suitable | Suitable | Not suitable |
| | | HPMC 2208 (100,000 cps, 20 mg) | | | |
| | Flowlac (100 mg) | | | | |
| | MCC pH102 (10 mg) | MCC pH 101 (60 mg) | | | |
| | | Carbomer (3 mg) | | | |
| | Aeroperl 300 (5 mg) | Poloxamer 407 (5 mg) | | | |
| | | Magnesium stearate (4 mg) | | | |
| | CL-PVP (10 mg) | | | | |
| F17 | Poloxamer (7 mg) | Aceclofenac (100 mg) | Suitable | Suitable | Not suitable |
| | Magnesium stearate (4 mg) | HPMC 2208 (100,000 cps, 20 mg) | | | |
| | | MCC pH 101 (60 mg) | | | |
| | | Carbomer (3 mg) | | | |
| | | Poloxamer 407 (6 mg) | | | |
| | | Magnesium stearate (4 mg) | | | |
| F18 | | Aceclofenac (100 mg) | Suitable | Suitable | Not suitable |
| | | HPMC 2208 (100,000 cps, 20 mg) | | | |
| | | MCC pH 101 (60 mg) | | | |
| | | Carbomer (3 mg) | | | |
| | | Poloxamer 407 (7 mg) | | | |
| | | Magnesium stearate (4 mg) | | | |
| F19 | Aceclofenac (100 mg) | Aceclofenac (100 mg) | Suitable | Suitable | Not suitable |
| | | HPMC 2208 (100,000 cps, 20 mg) | | | |
| | Flowlac (100 mg) | | | | |
| | MCC pH102 (10 mg) | MCC pH 101 (60 mg) | | | |
| | | Carbomer (3 mg) | | | |
| | Aeroperl 300 (5 mg) | Poloxamer 407 (6 mg) | | | |
| | | HPMC 2910 (5 mg) | | | |
| | CL-PVP (10 mg) | Magnesium stearate (4 mg) | | | |
| | Poloxamer (7 mg) | | | | |
| F20 | Magnesium stearate (4 mg) | Aceclofenac (100 mg) | Suitable | Suitable | Not suitable |
| | | HPMC 2208 (100,000 cps, 20 mg) | | | |
| | | MCC pH 101 (60 mg) | | | |
| | | Carbomer (3 mg) | | | |
| | | Poloxamer 407 (6 mg) | | | |
| | | HPMC 2910 (6 mg) | | | |
| | | Magnesium stearate (4 mg) | | | |
| F21 | | Aceclofenac (100 mg) | suitable | Suitable | Not suitable |
| | | HPMC 2208 (100,000 cps, 20 mg) | | | |
| | | MCC pH 101 (60 mg) | | | |
| | | Carbomer (3 mg) | | | |
| | | Poloxamer 407 (6 mg) | | | |
| | | HPMC 2910 (7 mg) | | | |
| | | Magnesium stearate (4 mg) | | | |
| F22 | Aceclofenac (95 mg) | Aceclofenac (105 mg) | Suitable | Suitable | Not suitable |
| | | HPMC 2208 (100,000 cps, 20 mg) | | | |
| | Flowlac (100 mg) | | | | |
| | MCC pH102 (10 mg) | MCC pH 101 (60 mg) | | | |
| | | Carbomer (3 mg) | | | |
| | Aeroperl 300 (5 mg) | Poloxamer 407 (6 mg) | | | |
| | | HPMC 2910 (5 mg) | | | |
| | CL-PVP (10 mg) | Magnesium stearate (4 mg) | | | |
| | Poloxamer (7 mg) | | | | |
| | Magnesium stearate (4 mg) | | | | |
| F23 | Aceclofenac (90 mg) | Aceclofenac (110 mg) | Suitable | Suitable | Not suitable |
| | | HPMC 2208 (100,000 cps, 20 mg) | | | |
| | Flowlac (100 mg) | | | | |
| | MCC pH102 (10 mg) | MCC pH 101 (60 mg) | | | |
| | | Carbomer (3 mg) | | | |
| | Aerosil 300 (5 mg) | Poloxamer 407 (6 mg) | | | |
| | CL-PVP (10 mg) | HPMC 2910 (6 mg) | | | |
| | Poloxamer (7 mg) | Magnesium stearate (4 mg) | | | |
| | Magnesium stearate (4 mg) | | | | |
| F24 | Aceclofenac (85 mg) | Aceclofenac (115 mg) | Suitable | Suitable | Not suitable |
| | | HPMC 2208 (100,000 cps, 20 mg) | | | |
| | Flowlac (100 mg) | | | | |
| | MCC pH102 (10 mg) | MCC pH 101 (60 mg) | | | |
| | | Carbomer (3 mg) | | | |
| | Aeroperl 300 (5 mg) | Poloxamer 407 (6 mg) | | | |
| | | HPMC 2910 (7 mg) | | | |
| | CL-PVP (10 mg) | Magnesium stearate (4 mg) | | | |
| | Poloxamer (7 mg) | | | | |
| | Magnesium stearate (4 mg) | | | | |
| F25 | Aceclofenac (115 mg) | Aceclofenac (85 mg) | Suitable | Suitable | Not suitable |
| | | HPMC 2208 (100,000 cps, 20 mg) | | | |
| | Flowlac (85 mg) | | | | |
| | MCC pH102 (24 mg) | MCC pH 101 (60 mg) | | | |
| | | Carbomer (3 mg) | | | |
| | Aeroperl 300 (10 mg) | Poloxamer 407 (6 mg) | | | |
| | | HPMC 2910 (5 mg) | | | |
| | CL-PVP (8 mg) | Magnesium stearate (4 mg) | | | |
| | Poloxamer (10 mg) | | | | |
| | Magnesium stearate (4 mg) | | | | |
| F26 | Aceclofenac (118 mg) | Aceclofenac (90 mg) | Suitable | Suitable | Not suitable |
| | | HPMC 2208 (100,000 cps, 20 mg) | | | |
| | Flowlac (85 mg) | | | | |
| | MCC pH102 (24 mg) | MCC pH 101 (60 mg) | | | |
| | | Carbomer (3 mg) | | | |
| | Aeroperl 300 (10 mg) | Poloxamer 407 (6 mg) | | | |
| | | HPMC 2910 (6 mg) | | | |
| | CL-PVP (8 mg) | Magnesium stearate (4 mg) | | | |
| | Poloxamer (10 mg) | | | | |
| | Magnesium stearate (4 mg) | | | | |
| F27 | Aceclofenac (105 mg) | Aceclofenac (95 mg) | Suitable | Suitable | Not suitable |
| | | HPMC 2208 (100,000 cps, 20 mg) | | | |
| | Flowlac (85 mg) | | | | |
| | MCC pH102 (24 mg) | MCC pH 101 (60 mg) | | | |
| | | Carbomer (3 mg) | | | |
| | Aeroperl 300 (10 mg) | Poloxamer 407 (6 mg) | | | |
| | | HPMC 2910 (7 mg) | | | |
| | CL-PVP (8 mg) | Magnesium stearate (4 mg) | | | |
| | Poloxamer (10 mg) | | | | |
| | Magnesium stearate (4 mg) | | | | |
| F28 | Aceclofenac (95 mg) | Aceclofenac (105 mg) | Suitable | Suitable | Suitable |
| | | HPMC 2208 (100,000 cps, 22 mg) | | | |
| | Flowlac (85 mg) | | | | |
| | MCC pH102 | MCC pH 101 (60 mg) | | | |
| | (24 mg) | Carbomer (3 mg) | | | |
| | Aeroperl 300 (10 mg) | Poloxamer 407 (6 mg) | | | |
| | | HPMC 2910 (5 mg) | | | |
| | CL-PVP (8 mg) | Magnesium stearate (4 mg) | | | |
| | Poloxamer (10 mg) | | | | |
| | Magnesium stearate (4 mg) | | | | |
| F29 | Aceclofenac (95 mg) | Aceclofenac (105 mg) | Suitable | Suitable | Suitable |
| | | HPMC 2208 (100,000 cps, 24 mg) | | | |
| | Flowlac (85 mg) | | | | |
| | MCC pH102 (24 mg) | MCC pH 101 (60 mg) | | | |
| | | Carbomer (3 mg) | | | |
| | Aeroperl 300 (10 mg) | Poloxamer 407 (6 mg) | | | |
| | | HPMC 2910 (6 mg) | | | |
| | CL-PVP (8 mg) | Magnesium stearate (4 mg) | | | |
| | Poloxamer (10 mg) | | | | |
| | Magnesium stearate (4 mg) | | | | |
| F30 | Aceclofenac (95 mg) | Aceclofenac (105 mg) | Suitable | Suitable | Suitable |
| | | HPMC 2208 (100,000 cps, 26 mg) | | | |
| | Flowlac (85 mg) | | | | |
| | MCC pH102 (24 mg) | MCC pH 101 (60 mg) | | | |
| | | Carbomer (3 mg) | | | |
| | Aeroperl 300 (10 mg) | Poloxamer 407 (6 mg) | | | |
| | | HPMC 2910 (7 mg) | | | |
| | CL-PVP (8 mg) | Magnesium stearate | | | |
| | Poloxamer (10 mg) | (4 mg) | | | |
| | Magnesium stearate (4 mg) | | | | |

### [Examples 1 to 14] Determination of Formulation of Preparation

A mixture of components for the immediate-release layer according to a formulation to be shown in Table 6 was primarily compressed to obtain a tablet having a hardness of approximately 2 to 3 kp, components for the slow-release layer were filled after the primary compression, and secondary compression was performed to obtain a tablet having a hardness of approximately 8 to 12 kp, thereby preparing a bilayered tablet. The prepared specimen was subjected to a dissolution test *in vitro* by the method to be described below.

A formulation of the slow-release part was determined as F30 in Experimental Example 4, and, Experimental Example 5 was performed to determine a detailed content of the excipient. It could be found that, when a slow-release layer and an immediate-release layer were prepared together at the same time, HPMC2208 used alone showed a more preferable dissolution profile than a mixture of HPMC2208 and HPMC2910, which were used as a slow-release base, in the slow-release part. Moreover, when PVP K30, which was used as a water-soluble additive, was added, an even more preferable dissolution profile was obtained. In addition, a more preferable dissolution profile was obtained from a bilayered tablet composed of a slow-release part and an immediate-release part, which contained Flowlac alone in the formulation thereof determined in Experimental Example 3.

### -Dissolution Rate Test

### Specimen: Bilayered tablets prepared in Examples

Liquid for Dissolution Test: Second liquid (pH 6.8) used in Disintegration Test according to the Korean Pharmacopoeia, 900 ml, 37 ± 0.5 °C

Dissolution Method: Second Method (paddle method) used in Dissolution Test according to the Korean Pharmacopoeia, 50 cycles per minute

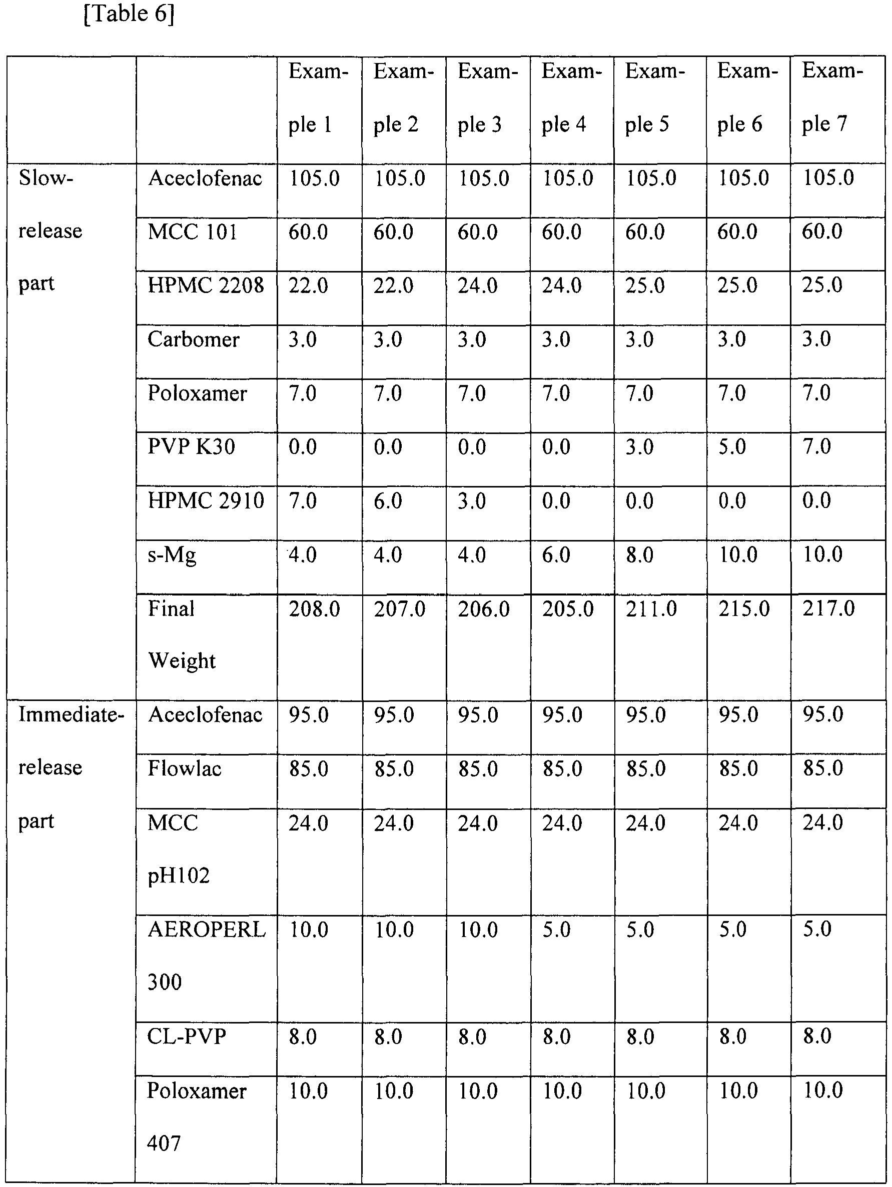

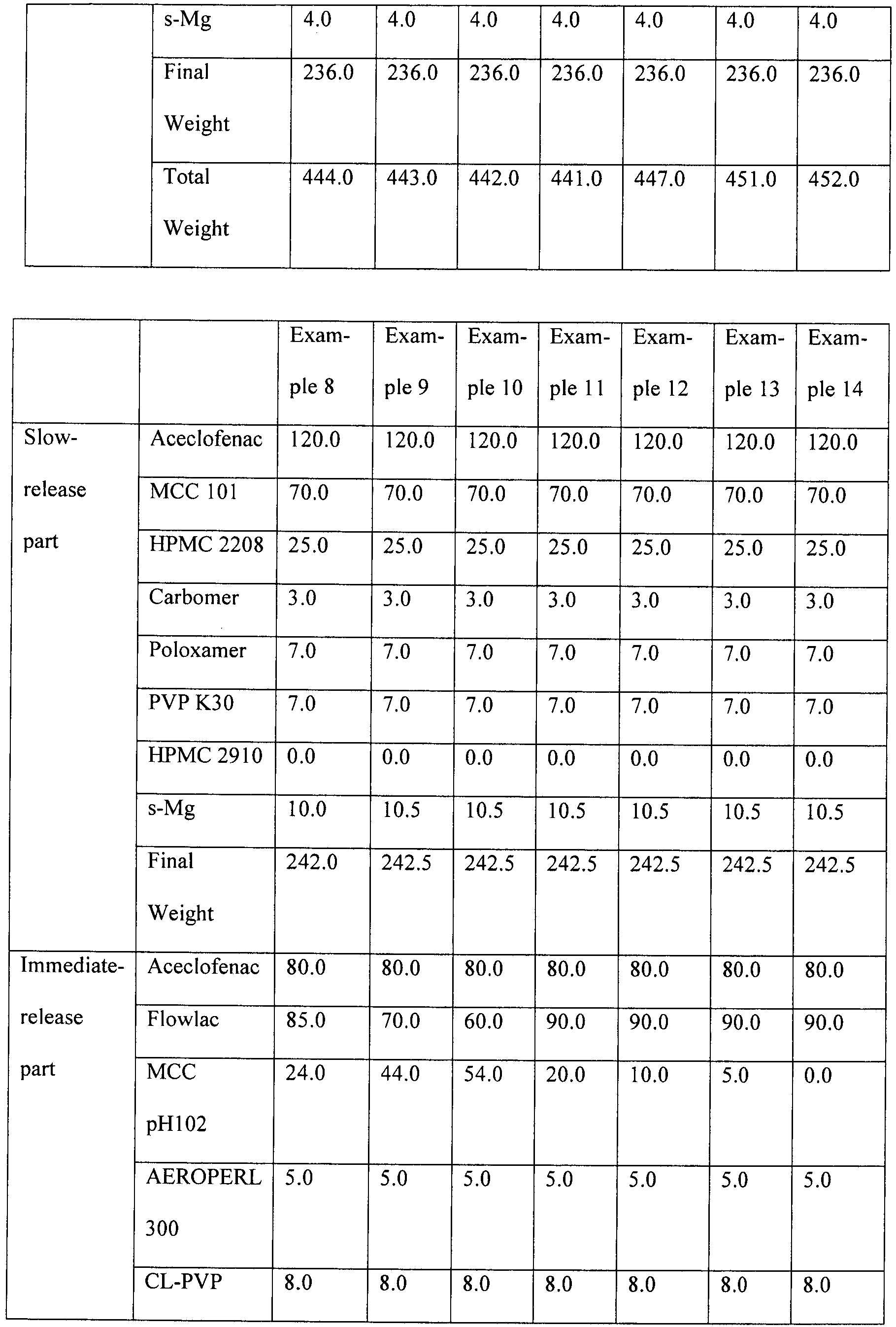

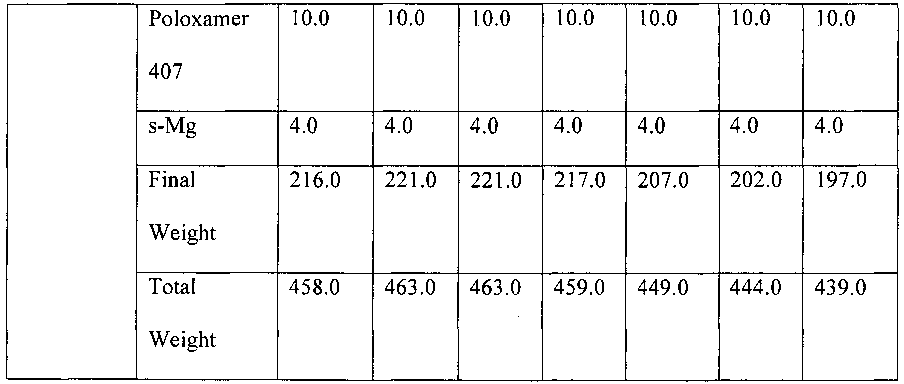

### [Experimental Example 5] Confirmation of Dissolution Profiles According to Contents of HPMC and Carbomer

A bilayered tablet was prepared by compressing components mixed in a ratio of immediate- and slow-release layers according to a composition shown in Table 7 to have a hardness of approximately 8 to 12 kp. The prepared specimen was subjected to an *in-vitro* dissolution rate test according to the method to be described below. The test results confirm that a dissolution profile of aceclofenac is gradually decreased as the contents of a pH-independent polymer base, that is HPMC2208, increase in the slow-release part. It was determined that, among Examples, Example 2 was suitable in that the dissolution profile of aceclofenac was uniformly and consistently shown for 24 hours in the content of HPMC2208 used in Example 2. The change in dissolution rate of the formulation in Example 2 according to the increase in the content of another carbomer, which was a slow-release base, was observed, and therefore it was determined that Examples 7 and 8 showing a significant difference from Example 2 were suitable in terms of the dissolution profile of aceclofenac according to the carbomer weight.

### -Dissolution Rate Test

### Specimen: Bilayered tablets prepared in Examples

Liquid for Dissolution Test: Second liquid (pH 6.8) used in Disintegration Test according to the Korean Pharmacopoeia, 900 ml, 37 ± 0.5 °C

Dissolution Method: Second Method (paddle method) used in Dissolution Test according to the Korean Pharmacopoeia, 50 cycles per minute

Dissolution Profiles According to HPMC Contents

**[Table 7]**

| | Components | Example 1 | | Example 2 | | Example 3 | | Example 4 | | Example 5 | |
|---|---|---|---|---|---|---|---|---|---|---|---|
| | | Content (g) | Content Ratio (%) | Content (g) | Content Ratio (%) | Content (g) | Content Ratio (%) | Content (g) | Content Ratio (%) | Content (g) | Content Ratio (%) |
| Immediate-Release Layer | Aceclofenac | 80.00 | 19.6 | 80.00 | 18.5 | 80.00 | 17.8 | 80.00 | 17.1 | 80.00 | 16.0 |
| | MCC 101 | 70.9 | 17.3 | 70.9 | 16.4 | 70.9 | 15.8 | 70.9 | 15.1 | 70.9 | 14.2 |
| | PVP K30 | 9.4 | 2.3 | 9.4 | 2.2 | 9.4 | 2.1 | 9.4 | 2.0 | 9.4 | 1.9 |
| | Croscarmellose sodium (post) | 4.8 | 1.2 | 4.8 | 1.1 | 4.8 | 1.1 | 4.8 | 1.0 | 4.8 | 1.0 |
| | Croscarmellose sodium (post) | 1.5 | 0.4 | 1.5 | 0.3 | 1.5 | 0.3 | 1.5 | 0.3 | 1.5 | 0.3 |
| | Magnesium stearate | 4.80 | 1.2 | 4.80 | 1.1 | 4.80 | 1.1 | 4.80 | 1.0 | 4.80 | 1.0 |
| Slow-Release Layer | Aceclofenac | 120.00 | 29.6 | 120.00 | 27.7 | 120.00 | 26.7 | 120.00 | 25.6 | 120.00 | 24.1 |
| | HPMC (100,000 cps) | 30.00 | 7.3 | 53.00 | 12.2 | 70.00 | 15.6 | 90.00 | 19.2 | 120.00 | 24.1 |
| | Carbomer | 0 | 0 | 0 | 0 | 0 | 0 | 0 | 0 | 0 | 0 |
| | MCC 101 | 70.00 | 17.1 | 70.00 | 16.2 | 70.00 | 15.6 | 70.00 | 14.9 | 70.00 | 14.0 |
| | PVP K30 | 7.00 | 1.7 | 7.00 | 1.6 | 7.00 | 1.6 | 7.00 | 1.5 | 7.00 | 1.4 |
| | Magnesium stearate | 10.40 | 2.5 | 10.40 | 2.4 | 10.40 | 2.3 | 10.40 | 2.2 | 10.40 | 2.1 |
| Total Weight | | 408.8 | 100.00 | 432.8 | 100.00 | 448.8 | 100.00 | 468.8 | 100.00 | 498.8 | 100.00 |

| | | | | | | | | | | | |
|---|---|---|---|---|---|---|---|---|---|---|---|
| Dissolution Profiles According to Carbomer Contents | | | | | | | | | | | |

**[Table 8]**

| | Components | Example 1 | | Example 2 | | Example 3 | | Example 4 | | Example 5 | |
|---|---|---|---|---|---|---|---|---|---|---|---|
| | | Content (g) | Content Ratio (%) | Content (g) | Content Ratio (%) | Content (g) | Content Ratio (%) | Content (g) | Content Ratio (%) | Content (g) | Content Ratio (%) |
| Immediate-Release Layer | Aceclofenac | 80.00 | 18.5 | 80.00 | 18.4 | 80.00 | 18.4 | 80.00 | 18.4 | 80.00 | 18.3 |
| | MCC 101 | 70.9 | 16.4 | 70.9 | 16.3 | 70.9 | 16.3 | 70.9 | 16.3 | 70.9 | 16.2 |
| | PVP K30 | 9.4 | 2.2 | 9.4 | 2.2 | 9.4 | 2.2 | 9.4 | 2.2 | 9.4 | 2.2 |
| | Croscarmellose sodium (post) | 4.8 | 1.1 | 4.8 | 1.1 | 4.8 | 1.1 | 4.8 | 1.1 | 4.8 | 1.1 |
| | Croscarmellose sodium (post) | 1.5 | 0.3 | 1.5 | 0.3 | 1.5 | 0.3 | 1.5 | 0.3 | 1.5 | 0.3 |
| | Magnesium stearate | 4.80 | 1.1 | 4.80 | 1.1 | 4.80 | 1.1 | 4.80 | 1.1 | 4.80 | 1.1 |
| Slow-Release Layer | Aceclofenac | 120.00 | 27.7 | 120.00 | 27.7 | 120.00 | 27.6 | 120.00 | 27.5 | 120.00 | 27.5 |
| | HPMC (100,000 cps) | 53.00 | 12.2 | 53.00 | 12.2 | 53.00 | 12.2 | 53.00 | 12.2 | 53.00 | 12.1 |
| | Carbomer | 1.00 | 0.2 | 2.00 | 0.5 | 3.00 | 0.7 | 4.00 | 0.9 | 5.00 | 1.1 |
| | MCC 101 | 70.00 | 16.2 | 70.00 | 16.1 | 70.00 | 16.16 | 70.00 | 16.1 | 70.00 | 16.0 |
| | PVP K30 | 7.00 | 1.6 | 7.00 | 1.6 | 7.00 | 1.6 | 7.00 | 1.6 | 7.00 | 1.6 |
| | Magnesium stearate | 10.40 | 2.4 | 10.40 | 2.4 | 10.40 | 2.4 | 10.40 | 2.4 | 10.40 | 2.4 |
| Total Weight | | 432.8 | 100.00 | 433.8 | 100.00 | 434.8 | 100.00 | 435.8 | 100.00 | 436.8 | 100.00 |

### [Experimental Example 6] Confirmation of Dissolution Profiles According to Contents Ratio of Immediate- and Slow-Release Layers

A bilayered tablet was prepared by preparing respective mixed granules of immediate- and slow-release layer parts as described in Examples 1 to 14, thereby mixing components of immediate- and slow-release layers according to a formulation shown in Table 9, and compressing the mixed components into a tablet to have a hardness of approximately 8 to 12 kp. The compressed specimen was subjected to an in-vitro dissolution rate test according to the test method to be described below. The test was performed to observe a dissolution rate according to a ratio of the contents of aceclofenac in the immediate- and slow-release layers based on Example 8 in Table 8. A formulation showing the early dissolution rate and 24-hour uniform dissolution profile of aceclofenac had a ratio of aceclofenac in the slow- and immediate-release parts of 3:1 to 1:1, and preferably 1.3:1. That is, it seems that the most suitable formulation is Sample 4 in Table 4.

### -Dissolution Rate Test

Specimen: Bilayered tablets prepared in Examples
Liquid for Dissolution Test: Second liquid (pH 6.8) used in Disintegration Test according to the Korean Pharmacopoeia, 900 ml, 37 ± 0.5 °C
Dissolution Method: Second Method (paddle method) used in Dissolution Test according to the Korean Pharmacopoeia, 50 cycles per minute

**[Table 9]**

| | | S1 | S2 | S3 | S4 |
|---|---|---|---|---|---|
| Slow- | Aceclofenac | 150 | 135 | 120 | 115 |
| Release Part | MCC 101 | 74.8 | 67.3 | 59.8 | 57.5 |
| | HPMC 2208 | 66.3 | 59.7 | 53 | 51 |
| | Carbomer | 3.6 | 3.3 | 2.9 | 2.8 |
| | Poloxamer | 0 | 0 | 0 | 0 |
| | PVP K30 | 8.7 | 7.84 | 7 | 6.7 |
| | HPMC 2910 | 0 | 0 | 0 | 0 |
| | s-Mg | 13 | 11.7 | 10.4 | 10 |
| | Total Weight | 316.4 | 284.84 | 253.1 | 243 |
| Immediate-Release Part | Aceclofenac | 50 | 65 | 80 | 85 |
| | MCC 101 | 51 | 65.7 | 70.9 | 86.45 |
| | PVP K30 | 6.8 | 8.7 | 9.4 | 11.5 |
| | NaCroscarmellose (Pre-mix) | 3.4 | 4.4 | 4.8 | 5.8 |
| | NaCroscarmellose (Post-mix) | 1.1 | 1.5 | 1.5 | 1.9 |
| | Poloxamer 407 | 0 | 0 | 0 | 0 |
| | s-Mg | 3.4 | 4.6 | 4.8 | 5.8 |
| | Total Weight | 115.7 | 149.9 | 171.4 | 196.45 |
| Total Weight | | 432.1 | 434.74 | 424.5 | 439.45 |

### [Experimental Example 7] Confirmation of Dissolution Profiles According to Amount of Solubilizer Used

A bilayered tablet was prepared by mixing components according to an amount of a solubilizer used according to a formulation shown in Table 10 and compressing the mixed components into a tablet to have a hardness of approximately 8 to 12 kp. The compressed specimen was subjected to a dissolution rate test *in vitro* according to the test method to be described below. To confirm the most ideal early dissolution rate of aceclofenac, the change in dissolution rate was observed by increasing an amount of the solubilizer. As a solubilizer, poloxamer which increases solubility of aceclofenac and generates smaller amounts of softening substances was used, and it was confirmed that the early dissolution rate of aceclofenac was increased with an increasing content of poloxamer. However, it can be noted through the following test that when the content of poloxamer increased to a certain level, and specifically, a ratio of a poloxamer content based on total parts by weight of the tablet was beyond 37.5:1, the early dissolution rate of aceclofenac no longer increased.

### -Dissolution Rate Test

Specimen: Bilayered tablets prepared in Examples
Liquid for Dissolution Test: Second liquid (pH 6.8) used in Disintegration Test according to the Korean Pharmacopoeia, 900 ml, 37 ± 0.5 °C
Dissolution Method: Second Method (paddle method) used in Dissolution Test according to the Korean Pharmacopoeia, 50 cycles per minute

**[Table 10]**

| | | S4 | S5 | S6 | S7 |
|---|---|---|---|---|---|
| Slow-Release Part | Aceclofenac | 115 | 115 | 115 | 115 |
| | MCC 101 | 57.5 | 57.5 | 57.5 | 57.5 |
| | HPMC 2208 | 51 | 51 | 51 | 51 |
| | Carbomer | 2.8 | 2.8 | 2.8 | 2.8 |
| | Poloxamer | 0 | 3 | 6 | 9 |
| | PVP K30 | 6.7 | 6.7 | 6.7 | 6.7 |
| | HPMC 2910 | 0 | 0 | 0 | 0 |
| | s-Mg | 10 | 10 | 10 | 10 |
| | Total Weight | 243 | 246 | 249 | 252 |
| Immediate-Release Part | Aceclofenac | 85 | 85 | 85 | 85 |
| | MCC 101 | 86.45 | 86.45 | 86.45 | 86.45 |
| | PVP K30 | 11.5 | 11.5 | 11.5 | 11.5 |
| | NaCroscarmellose (Pre-mix) | 5.8 | 5.8 | 5.8 | 5.8 |
| | NaCroscarmellose (Post-mix) | 1.9 | 1.9 | 1.9 | 1.9 |
| | Poloxamer 407 | 0 | 3 | 6 | 9 |
| | s-Mg | 5.8 | 5.8 | 5.8 | 5.8 |
| | Total Weight | 194.45 | 199.45 | 202.45 | 205.45 |
| Total Weight | | 439.45 | 445.45 | 451.45 | 457.45 |

### [Experimental Example 8] Confirmation of Dissolution Profiles According to pH

A bilayered tablet was prepared by preparing mixed granules of immediate-and slow-release layers according to Sample 6 tested in Experimental Example 7, and compressing the mixed granules into a tablet. The compressed specimen and the commercially-available tablet, Airtal™ (as an aceclofenac 100 mg/tablet, Daewoong Pharmaceutical Co., Ltd.), were subjected to an *in vitro* test according to the test method to be described below so as to confirm the dissolution profiles of an aceclofenac slow-release tablet according to pH change. It was observed that dissolution was consistently and uniformly shown regardless of pH as Sample 6 of the present invention used poloxamer as a solubilizer, a carbomer as a pH-dependent polymer base, and HPMC2208 as a pH-independent polymer base. Moreover, the dissolution profile of one Airtal™ tablet, which was commercially available as in Comparative Example, was confirmed together with the dissolution profile of Sample 6, and it was clearly confirmed that there were differences in dissolution profiles between the specimen of Sample 6 and Airtal™. Airtal™ showed a low dissolution profile at pH 1.2, and a high dissolution profile at pH 6.8, which was the same as the physical properties of aceclofenac, unlike the specimen of Sample 6. On the other hand, the specimen of Sample 6 showed a higher dissolution rate of 10% than Airtal™, which was used as Comparative Example, at pH 1.2 and pH 4.0. From such a result, the specimen of Sample 6 was expected to show higher absorption than Comparative Example, Airtal™, in a living body, which is under an acidic condition. In addition, the specimen of Sample 6 showed more uniformly and consistently maintained dissolution profiles than Comparative Example, Airtal™, at pH 6.8 and in water, and therefore the bioavailability of aceclofenac was expected to be consistently shown for 24 hours.

### -Dissolution Rate Test

Specimen: Bilayered tablets prepared in Examples
Liquid for Dissolution Test: Second liquid (pH 6.8) used in Disintegration Test according to the Korean Pharmacopoeia, 900 ml, 37 ± 0.5 °C
Dissolution Method: Second Method (paddle method) used in Dissolution Test according to the Korean Pharmacopoeia, 50 cycles per minute

### [Experimental Example 9] Pre-Clinical Test According to Amount of Solubilizer Used

Pharmacokinetics (PKs) of aceclofenac according to an amount of a solubilizer (a degree of solubilization) were confirmed by the pre-clinical test to be described below with the aceclofenac slow-release tablet of Sample 6 in Experimental Example 7. When the PKs of aceclofenac were confirmed with the Airtal™ tablet of Comparative Example, and Sample 6 from a beagle dog according to the following test method, it could be confirmed that, like the *in-vitro* results, Cₘₐₓ increased and tₘₐₓ of aceclofenac was delayed due to the slow-release preparation as the content of the solubilizer, poloxamer, was increased to a certain level. This was expected as the result corresponding to the solution of the technical problems of the present invention, and optimum effects were confirmed from a clinical test, which is the final goal of the present invention, based on the above-described test.

### -Test Method

A test substance (aceclofenac 200 mg) and 100 mg of commercially available Airtal™ were orally administered one by one in a dosage form of tablets to each of the beagle dogs (n=6), and a blood sample was taken from each dog at predetermined times to separate the serum. Subsequently, the concentration of aceclofenac in the serum of the beagle dog was measured. As pharmacokinetic parameters, AUCₜ (blood concentration from administration time (t) to measure final blood concentration-area under the time curve), AUC_{∞} (blood concentration from administration time to infinite time-area under the time curve), Cₘₐₓ (maximum blood concentration), tₘₐₓ (time to maximum blood concentration), and t_{1/2} (elimination half-life in blood) were estimated using the BA Calc 2007 program. Values obtained by dividing AUC_{∞} and Cₘₐₓ by dose contents were represented as AUCi/doseC and max/dose, and a ratio of AUCt and AUCi was represented as AUCt/AUCi. Significances between substances were confirmed by a Student's t-test with a confidence interval of 95%, and the results are shown in Table 9.

### [Experimental Example 10] Clinical Test According to Optimized Formulation of Preparation

To evaluate pharmacodynamic equivalence between Airtal™ (as an aceclofenac 100 mg tablet, Daewoong Pharmaceutical Co., Ltd.) commercially available as an aceclofenac tablet and the specimen of Sample 6 in Experimental Example 7, 42 healthy male volunteers between the ages of 20 and 55 selected according to selection and exclusion standards for clinical test proposals were divided into 6 groups having 7 persons in each, and subjected to randomization, publicization, single administration, and a 3-treatment, 3-period Williams' design clinical trial under the following conditions in first, second and third periods. The results are shown in Table 11.

### -Test Conditions

Specimen: Experimental Example 7 and Airtal™ Tablet
Subjects per Group: 6 groups having 7 persons each, total 42 persons

**[Table 11]**

| Group | No. of Subjects (persons) | First Period | Second Period | Third Period |
|---|---|---|---|---|
| A | 7 | Airtal™ (empty stomach) | Experimental Example 7 (empty stomach) | Experimental Example 7 (after meals) |
| B | 7 | Experimental Example 7 (after meals) | Airtal™ (empty stomach) | Experimental Example 7 (empty stomach) |
| C | 7 | Experimental Example 7 (empty stomach) | Experimental Example 7 (after meals) | Airtal™ (empty stomach) |
| D | 7 | Experimental Example 7 (after meals) | Experimental Example 7 (empty stomach) | Airtal™ (empty stomach) |
| E | 7 | Experimental Example 7 (empty stomach) | Airtal™ (empty stomach) | Experimental Example 7 (after meals) |
| F | 7 | Airtal™ (empty stomach) | Experimental Example 7 (after meals) | Experimental Example 7 (empty stomach) |

### Administration:

On the administration day (1d) of the first period, one tablet of Airtal™ produced by Daewoong Pharmaceutical Co., Ltd. was administered to a subject of Group A twice a day on an empty stomach, one tablet of Experimental Example 7 was administered to a subject of Group B once a day after meals, one tablet of Experimental Example 7 was administered to a subject of Group C once a day on an empty stomach, one tablet of Experimental Example 7 was administered to a subject of Group D once a day after meals, one tablet of Experimental Example 7 was administered to a subject of Group E once a day on an empty stomach, and one tablet of Airtal™ produced by Daewoong Pharmaceutical Co., Ltd. was administered to a subject of Group F twice a day on an empty stomach.

After a 7-day withdrawal period, on the administration day of the second period (8d), one tablet of Experimental Example 7 was administered to a subject of Group A once a day on an empty stomach, one tablet of Airtal™ produced by Daewoong Pharmaceutical Co., Ltd. was administered to a subject of Group B twice a day on an empty stomach, one tablet of Experimental Example 7 was administered to a subject of Group C twice a day after meals, one tablet of Experimental Example 10 was administered to a subject of Group D once a day on an empty stomach, one tablet of Airtal™ produced by Daewoong Pharmaceutical Co., Ltd. was administered to a subject of Group E twice a day on an empty stomach, and one tablet of Experimental Example 7 was administered to a subject of Group F once a day after meals.

After a 7-day withdrawal period, on the administration day of the third period (15d), one tablet of Experimental Example 7 was administered to a subject of Group A once a day after meals, one tablet of Experimental Example 7 was administered to a subject of Group B once a day on an empty stomach, one tablet of Airtal™ produced by Daewoong Pharmaceutical Co., Ltd. was administered to a subject of Group C twice a day on an empty stomach, one tablet of Airtal™ produced by Daewoong Pharmaceutical Co., Ltd. was administered to a subject of Group D twice a day on an empty stomach, one tablet of Experimental Example 7 was administered to a subject of Group E once a day after meals, and one tablet of Experimental Example 7 was administered to a subject of Group F once a day on an empty stomach.

### Time for Taking Blood Sample:

Airtal™: Pre-dose (0h), 0.5, 1, 1.5, 2, 2.5, 3, 4, 6, 8, 10, 12, 12,5, 13, 13.5, 14, 14.5, 15, 16, 18, 20, and 24 h for each period (total 22 times)
Experimental Example 7: Pre-dose (0h), 0.5, 1, 1.5, 2, 2.5, 3, 3.5, 4, 5, 6, 8, 10, 12, 16, 20, and 24 h for each period (total 17 times)

### Evaluation of Pharmacokinetic Characteristics:

After Airtal™ produced by Daewoong Pharmaceutical Co., Ltd. and the tablet of Experimental Example 7 were administered, blood aceclofenac concentrations were measured over 24 hours, and then pharmacokinetic parameters were evaluated as follows:
Evaluation Parameters:
First Evaluation Parameters - Cₘₐₓ, AUCₜ (t=24)

### Second Evaluation Parameters - tₘₐₓ, t_{1/2}, AUC_{∞}

**[Table 12]**

| Pharmacokinetic Parameters | Mean±SD | | |
|---|---|---|---|
| | Airtal™ (empty stomach) | Experimental Example 7 (empty stomach) | Experimental Example 7 (after meals) |
| Cmax (µg/mL) | 10.26±1.81 | 10.61±2.07 | 10.34±2.27 |
| AUC₂₄(h*µg/mL) | 42.33±8.35 | 41.05±7.66 | 41.61±7.71 |
| AUC_{∞}(h*µg/mL) | 43.22±8.62 | 43.31±7.89 | 43.22±7.69 |
| Tₘₐₓ(h) | 1.7±0.7 | 1.5±0.7 | 2.7±1.2 |
| λ_{z}(1/h) | 0.39±0.14 | 0.16±0.07 | 0.21±0.06 |
| t_{1/2}(h) | 2.1±0.9 | 5.0±2.1 | 3.9±2.5 |

As shown in Table 12, according to the test of pharmacodynamic equivalence between the bilayered tablet of Experimental Example 7 and Airtal™ commercially available as an aceclofenac tablet, it was revealed that both preparations expressed pharmacodynamically-equivalent effects with a confidence interval of 90%.

While the invention has been shown and described with reference to certain exemplary embodiments thereof, it will be understood by those skilled in the art that various changes in form and details may be made therein without departing from the scope of the invention as defined by the appended claims.

## Claims

1. An aceclofenac controlled-release preparation for oral administration, comprising:
an immediate-release layer including aceclofenac, a solubilizer, a water-soluble additive, a disintegrating agent, a filler and a fast-acting excipient; and
a slow-release layer including aceclofenac, a solubilizer and a base for release control,
wherein the base for release control uses a mixture of hydroxypropylmethylcellulose (HPMC) and a carbomer in a weight ratio of 1:1 to 30:1, and the preparation is administered once a day.

2. The preparation according to claim 1, wherein the HPMC has a viscosity of 80 to 120 Pa*s (80,000 to 120,000 cps).

3. The preparation according to claim 1, wherein the immediate-release layer includes aceclofenac in a content of 40 to 100 mg, and the slow-release layer includes aceclofenac in a content of 100 to 160 mg.

4. The preparation according to claim 1, wherein the preparation includes at least one compound selected from the group consisting of a poloxamer, organic acid and organic acid salt monoglyceride derivatives, a glyceride and a derivative thereof, diethylene glycol ester, sorbital ester, a carbonate salt and polyoxyethylene ether, Gelucire 44/14, sodium hydrocarbons of polyethyleneglycols, Brijs and Labrafils, sodium laurylsulfate, and a mixture thereof as a solubilizer.

5. The preparation according to claim 1, wherein the preparation has a controlled dissolution rate of 45 to 65% for 1 hour, 65 to 85% for 12 hours and 85% or more for 24 hours in an in-vitro test in which a paddle revolves at a rate of 50 rpm/min according to a general test method regulated by the Korean Pharmacopoeia, and a surface of the slow-release layer is coated with the immediate-release layer.

6. The preparation according to claim 5, wherein the preparation is a pellet-type tablet or multi tablet, which is prepared by mixing an immediate-release layer and a slow-release layer.

7. The preparation according to claim 5, wherein the preparation is a capsule prepared by filling a mixture of components for the immediate-release layer and the slow-release layer.

8. The preparation according to any one of claims 1 to 7, wherein the preparation shows a maximum blood concentration (Cₘₐₓ) of aceclofenac of 30 to 60 µg/ml, an area under a blood concentration versus time curve (AUCₜ) of 5 to 15 h*µg/ml, AVC_{∞} of 30 to 60 h* µg /ml, and a time to peak concentration (Tₘₐₓ) of 1.2 to 3 h in an *in-vivo* test using healthy persons.

## Patentansprüche

1. Eine Aceclofenac-Präparation zur kontrollierten Freisetzung für die orale Verabreichung, welche umfasst:
eine Schicht zur sofortigen Freisetzung, die Aceclofenac, einen Lösungsvermittler, ein wasserlösliches Additiv, ein Zerfallsmittel, ein Füllmittel und einen schnell wirkenden Exzipienten einschließt; und
eine Schicht zur langsamen Freisetzung, die Aceclofenac, einen Lösungsvermittler und eine Base zur Freisetzungskontrolle einschließt, worin bei der Base zur Freisetzungskontrolle eine Mischung von Hydroxypropylmethylcellulose (HPMC) und einem Carbomer in einem Gewichtsverhältnis von 1:1 bis 30:1 verwendet wird, und die Präparation einmal täglich verabreicht wird.

2. Präparation nach Anspruch 1, wobei das HPMC eine Viskosität von 80 bis 120 Pa*s (80.000 bis 120.000 cps) aufweist.

3. Präparation nach Anspruch 1, wobei die Schicht zur sofortigen Freisetzung Aceclofenac in einem Gehalt von 40 bis 100 mg einschließt und die Schicht zur langsamen Freisetzung Aceclofenac in einem Gehalt von 100 bis 160 mg einschließt.

4. Präparation nach Anspruch 1, wobei die Präparation mindestens eine Verbindung, welche aus der Gruppe bestehend aus einem Poloxamer, Monoglycerid-Derivaten einer organischen Säure und eines Salzes einer organischen Säure, einem Glycerid und einem Derivat davon, Diethylenglycolester, Sorbitanester, einem Carbonatsalz und Polyoxyethylenether, Natrium-Kohlenwasserstoffen von Polyethylenglycolen, Natriumlaurylsulfat und einer Mischung davon ausgewählt ist, als Lösungsvermittler einschließt.

5. Präparation nach Anspruch 1, wobei die Präparation eine kontrollierte Auflösungsrate von 45 bis 65 % innerhalb einer Stunde, 65 bis 85 % innerhalb von 12 Stunden und 85 % oder mehr innerhalb von 24 Stunden in einem *in-vitro-Test,* bei dem sich ein Paddel mit einer Rate von 50 Umdrehungen pro Minute dreht, gemäß einem allgemeinen Testverfahren entsprechend den Bestimmungen der koreanischen Pharmakopoe, aufweist und eine Oberfläche der Schicht zur langsamen Freisetzung mit der Schicht zur sofortigen Freisetzung beschichtet ist.

6. Präparation nach Anspruch 5, wobei die Präparation eine Tablette von Pellet-Typ oder eine Multitablette ist, welche durch Mischen einer Schicht zur sofortigen Freisetzung und einer Schicht zur langsamen Freisetzung hergestellt wird.

7. Präparation nach Anspruch 5, wobei die Präparation eine Kapsel ist, welche durch Füllen einer Mischung von Komponenten für die Schicht zur sofortigen Freisetzung und für die Schicht zur langsamen Freisetzung hergestellt wird.

8. Präparation nach irgendeinem der Ansprüche 1 bis 7, wobei die Präparation in einem in-vivo-Test bei gesunden Personen eine maximale Blutkonzentration (Cₘₐₓ) von Aceclofenac von 30 bis 60 µg/ml, eine Fläche unter einer Blutkonzentrationgegen-Zeit-Kurve (AUCₜ) von 5 bis 15 h*µg/ml, eine AUC_{∞} von 30 bis 60 h*µg/ml und eine Zeit bis zur Gipfelkonzentration (Tₘₐₓ) von 1,2 bis 3 h zeigt.

## Revendications

1. Préparation à libération contrôlée d'acéclofénac pour une administration orale, comprenant :
une couche à libération immédiate incluant de l'acéclofénac, un solubilisant, un additif soluble dans l'eau, un agent délitant, une charge et un excipient à action rapide ; et
une couche à libération lente incluant de l'acéclofénac, un solubilisant et une base pour le contrôle de la libération,
dans laquelle la base pour le contrôle de la libération utilise un mélange d'hydroxypropylméthylcellulose (HPMC) et un carbomère dans un rapport en poids de 1 : 1 à 30 : 1, et la préparation est administrée une fois par jour.

2. Préparation selon la revendication 1, dans laquelle l'HPMC a une viscosité de 80 à 120 Pa*s (80 000 à 120 000 cps).

3. Préparation selon la revendication 1, dans laquelle la couche à libération immédiate inclut de l'acéclofénac à une teneur de 40 à 100 mg, et la couche à libération lente inclut de l'acéclofénac à une teneur de 100 à 160 mg.

4. Préparation selon la revendication 1, dans laquelle la préparation inclut au moins un composé choisi dans le groupe consistant en un poloxamère, un acide organique et des dérivés monoglycéride de sel d'acide organique, un glycéride et un dérivé de celui-ci, un ester de diéthylène glycol, un ester de sorbital, un ester de carbonate et du poly(oxyéthylèneéther), des hydrocarbures sodiques de poly(éthylèneglycols), du laurylsulfate de sodium et un mélange de ceux-ci comme solubilisant.

5. Préparation selon la revendication 1, dans laquelle la préparation a un taux de dissolution contrôlé de 45 à 65% pendant 1 heure, 65 à 85% pendant 12 heures et 85% ou plus pendant 24 heures dans un essai *in vitro* dans lequel une palette tourne à une vitesse de 50 tr/min selon un procédé d'essai général réglementé par la pharmacopée coréenne, et une surface de la couche à libération lente est enrobée avec la couche à libération immédiate.

6. Préparation selon la revendication 5, dans laquelle la préparation est un comprimé ou multicomprimé de type pilule, qui est préparé en mélangeant une couche à libération immédiate et une couche à libération lente.

7. Préparation selon la revendication 5, dans laquelle la préparation est une capsule préparée en y remplissant un mélange de composants pour la couche à libération immédiate et la couche à libération lente.

8. Préparation selon l'une quelconque des revendications 1 à 7, dans laquelle la préparation montre une concentration maximale dans le sang (Cₘₐₓ) en acéclofénac de 30 à 60 µg/mL, une aire sous la courbe de la concentration dans le sang en fonction du temps (ASCₜ) de 5 à 15 h*µg/mL, ASC_{∞} de 30 à 60 h* µg/mL, et un temps nécessaire pour atteindre la concentration maximale (Tₘₐₓ) de 1,2 à 3 h dans un essai *in vivo* employant des personnes en bonne santé.
